(19) Europäisches Patentamt
European Patent Office
Office européen des brevets

(11) EP 3 512 494 B1

(12) EUROPEAN PATENT SPECIFICATION

(45) Date of publication and mention of the grant of the patent:
**17.06.2026 Bulletin 2026/25**

(21) Application number: **17771971.3**

(22) Date of filing: **13.09.2017**

(51) International Patent Classification (IPC):
***A61K 8/92*** *(2006.01)* ***A61K 8/73*** *(2006.01)*
***C11D 7/26*** *(2006.01)* ***A61Q 5/02*** *(2006.01)*
***A61Q 19/10*** *(2006.01)*

(52) Cooperative Patent Classification (CPC):
**A61Q 5/02; A61K 8/737; A61K 8/922; A61Q 19/10;**
A61K 2800/30; A61K 2800/34

(86) International application number:
**PCT/US2017/051249**

(87) International publication number:
**WO 2018/052920 (22.03.2018 Gazette 2018/12)**

# (54) PERSONAL CARE COMPOSITION FORMED WITH GLYCERIDE ESTER CRYSTALS HAVING IMPROVED COACERVATE PROPERTIES

PFLEGEZUBEREITUNG GEBILDET AUS GLYCERIDESTERKRISTALE MIT VERBESSERTEN KOAZERVAT EIGENSCHAFTEN

COMPOSITION DE SOIN PERSONEL OBTENUE À PARTIR DE CRISTAUX D’ESTER GLYCERIDE AYANT DES PROPRIÉTÉS DE COACÉRVATION AMÉLIORÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.09.2016 US 201662393910 P**

(43) Date of publication of application:
**24.07.2019 Bulletin 2019/30**

(73) Proprietor: **The Procter & Gamble Company**
**Cincinnati, OH 45202 (US)**

(72) Inventors:
- **COCHRAN, Brooke, Michele**
**Cincinnati, Ohio 45202 (US)**
- **HUTTON, Howard, David, III**
**Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**EP-A1- 3 020 390**
**WO-A1-97/26854**
**US-A1- 2003 224 954**
**US-A1- 2010 322 878**
**US-A1- 2016 106 663**

- **"Herbal Essence Shampoo (Natural Supply) Product Description", MINTEL GNPD, 1 June 2004 (2004-06-01), Internet, pages 1 - 2, XP055114658, Retrieved from the Internet <URL:http://www.gnpd.com/sinatra/recordpage/277011/from_search/U5rPN4sfyn/with_sort/2/> [retrieved on 20140422]**

EP 3 512 494 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### FIELD OF THE INVENTION

The present disclosure generally relates to personal care compositions including glyceride ester crystals to provide improved coacervate properties.

### BACKGROUND OF THE INVENTION

Consumers desire personal care compositions which simultaneously exhibit multiple qualities such as good cleaning ability and excellent feel during use. Achieving an optimal selection of qualities requires personal care compositions to have variations in formulations and target specific combinations of qualities. For example, certain personal care compositions having a coacervate with excellent feel during use have included multiple cationic polymers. It would be advantageous to provide personal care compositions which can form a greater quantity of a coacervate and can provide improved coacervate properties such as improved lather creaminess and improved wet hair feel and detangling using new components and formulations.

US Patent Application US-A-81778610 discloses personal care compositions which comprise a synthetic polymer with a specified ratio of monomers that enhances coacervate formation, the size and the viscoelasticity of the coacervate results in improved deposition of cosmetic agents.

US Patent application US-A-2016/106663 discloses a method of making a personal care composition comprising the steps of preparing a premix composition by mixing from 0.30% to 4% of hydrogenated castor oil by weight, wherein the hydrogenated castor oil comprises particles of which 90% by weight of the total hydrogenated castor oil consists of particle with size of less than 60 micrometers, from 15% to 40% of one or more surfactants, from 60% to 80% of an aqueous carrier; heating the premix composition to 65-84° C; and then cooling the premix composition to a temperature of 60° C to 20° C.

### SUMMARY OF THE INVENTION

A personal care composition as defmed in claim 1 comprising: one or more surfactants, the one or more surfactants comprising one or more anionic surfactants, amphoteric surfactants, and zwitterionic surfactants; a cationic polymer; and glyceride ester crystals, and wherein the percentage of the personal care composition that participates in the coacervate phase at a 9:1 dilution is from about 30% to about 600% higher compared to a personal care composition without glyceride ester crystals.

The personal care composition includes about 10% to about 25%, by weight, of one or more anionic surfactants, about 0.01% to about 0.3%, by weight, of a cationic guar polymer, and about 0.01% to about 0.50%, by weight, trihydroxystearin. The cationic guar polymer has a weight average molecular weight of about 2 million g/mol or less. The personal care composition forms a greater quantity of coacervate than a similar personal care composition formed without glyceride ester crystals.

A method of making a personal care composition as defined in claim 10, which includes mixing one or more surfactants, a cationic polymer, and a liquid carrier to form a first mixture, and adding a glyceride ester to the first mixture to form a personal care composition. The one or more surfactants include one or more anionic surfactants, amphoteric surfactants, and zwitterionic surfactants. The personal care composition forms a greater quantity of coacervate than a similar personal care composition formed without glyceride ester crystals.

A personal care composition includes one or more surfactants, a cationic polymer, and glyceride ester crystals. The one or more surfactants include one or more anionic surfactants, amphoteric surfactants, and zwitterionic surfactants. The personal care composition having a light transmittance value at 400 nm of about 0.5% or more.

### DETAILED DESCRIPTION OF THE INVENTION

While the specification concludes with claims particularly pointing out and distinctly claiming the invention, it is believed that the present disclosure will be better understood from the following description.

As used herein, the term "fluid" includes liquids and gels.

As used herein, the articles including "a" and "an" when used in a claim, are understood to mean one or more of what is claimed or described.

As used herein, "comprising" means that other steps and other ingredients which do not affect the end result can be added. This term encompasses the terms "consisting of" and "consisting essentially of".

As used herein, "mixtures" is meant to include a simple combination of materials and any compounds that may result from their combination.

As used herein, "molecular weight" or "M.Wt." refers to the weight average molecular weight unless otherwise stated. Molecular weight is measured using industry standard method, gel permeation chromatography ("GPC").

As used herein, "personal care composition" includes products such as shampoos, conditioners, conditioning shampoos, shower gels, liquid hand cleansers, hair colorants, facial cleansers, laundry detergent, dish detergent, and other surfactant-based liquid compositions.

As used herein, the terms "include," "includes," and "including," are meant to be non-limiting and are understood to mean "comprise," "comprises," and "comprising," respectively.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Unless otherwise noted, all component or composition levels are in reference to the active portion of that component or composition, and are exclusive of impurities, for example, residual solvents or by-products, which may be present in commercially available sources of such components or compositions.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

## Personal Care Compositions

As will be described herein, personal care compositions are disclosed which exhibit improved coacervate properties including improved coacervate quantities and excellent wet feel characteristics during use. The personal care compositions can include at least glyceride ester crystals, a surfactant, a cationic polymer, and a liquid carrier.

Glyceride ester crystals can be useful to both improve the properties of a coacervate and increase the quantity of coacervate formed throughout the dilution profile of a personal care compositions. Glyceride ester crystals can also be useful to form a coacervate in personal care compositions which would otherwise be incapable of forming a coacervate. Without being bound by theory, it is theorized that glyceride ester crystals, such as trihydroxystearin crystals, can act as nucleation particles for the formation of a coacervate phase created by interactions between the surfactant and cationic polymer. It is also theorized that the addition of glyceride ester crystals can further modify the coacervate properties to be more appealing than any coacervate formed from the interaction of a surfactant and cationic polymer alone. As will be appreciated, these unexpected discoveries enable personal care compositions of suitable formulations to exhibit a variety of hereto unknown properties. For example, the use of glyceride ester crystals can facilitate the formation of increased quantities of a coacervate phase in personal care compositions, improve the feel of personal care compositions, and enable personal care compositions to exhibit desired light transmittances including the appearance of translucency to a consumer. The percentage of the personal care composition that participates in the coacervate phase at a 9:1 dilution is from about 30% to about 600% higher compared to a similar personal care composition without glyceride ester crystals; as measured by the Coacervate Centrifuge Method. Alternatively, the percentage of the personal care composition that participates in the coacervate phase at a 9:1 dilution is from about 50% to about 350% higher compared to a similar personal care composition without glyceride ester crystals; as measured by the Coacervate Centrifuge Method. It is also contemplated that the ratio of coacervate quantity to glyceride ester crystal level can be from about 15:1 to about 75:1 at a 9:1 dilution compared to a similar personal care composition without glyceride ester crystals.

As used herein, "translucent" means permitting some amount of visible light to transmit through an object, for example, a composition. Suitable light transmittance can be determined using a UV/Vis spectrometer. As used herein, suitable light transmittance can mean, about 0.5% or more light having a wavelength of 400 nm can transmit through a standard sample, alternatively, about 1% or more light having a wavelength of 400 nm can transmit through a standard sample, alternatively, about 5% or more light having a wavelength of 400 nm can transmit through a standard sample, alternatively, about 15% or more light having a wavelength of 400 nm can transmit through a standard sample, alternatively, about 25% or more light having a wavelength of 400 nm can transmit through a standard sample, alternatively, about 40% or more light having a wavelength of 400 nm can transmit through a standard sample, alternatively, about 55% or more light having a wavelength of 400 nm can transmit through a standard sample, and alternatively, about 65% or more light having a wavelength of 400 nm can transmit through a standard sample. If substantially all visible light transmits through an object, this shall be referred to as "clear." An opaque solution can mean about 0% of light having a wavelength of 400 nm can transmit through a standard sample. As can be appreciated, translucent or clear personal care compositions can be formed by selecting components which are translucent or clear after dissolving the components in the liquid carrier of a personal care composition.

## A. Glyceride Ester Crystals

Traditionally glyceride ester compounds were used as a structurant for personal care compositions. For example, Thixcin® R is trihydroxystearin, a commercial hydrogenated castor oil produced by Elementis Specialties of New Jersey, and marketed as a stabilizer and structurant for personal care compositions. Suitable glyceride esters for the personal care compositions described herein can be selected from any crystallizable glyceride esters which can allow for the formation of a coacervate in personal care compositions including a suitable surfactant and a cationic polymer. For example, suitable glyceride esters are hydrogenated castor oils such as trihydroxystearin or dihydroxystearin.

Examples of additional crystallizable glyceride esters can include the substantially pure triglyceride of 12-hydroxystearic acid. 12-hydroxystearic acid is the pure form of a fully hydrogenated triglyceride of 12-hydrox-9-cis-octadecenoic acid. As can be appreciated, many additional glyceride esters are possible. For example, variations in the hydrogenation process and natural variations in castor oil can enable the production of additional suitable glyceride esters from castor oil.

Suitable glyceride esters can also be formed from mixtures of one or more glycerides. For example, a mixture of glycerides including about 80% or more, by weight of the mixture, castor oil, can be suitable. Other suitable mixtures can include mixtures of only triglycerides, mixtures of diglycerides and triglycerides, mixtures of triglycerides with diglycerides and limited amounts, e.g., less than about 20%, by weight of the mixture, of monoglyerides; or any mixture thereof which includes about 20% or less, by weight of the mixture, of a corresponding acid hydrolysis product of any of the glycerides. About 80% or more, by weight of a mixture, can be chemically identical to a glyceride of fully hydrogenated ricinoleic acid, i.e., glyceride of 12-hydroxystearic acid. Hydrogenated castor oil can be modified such that in a given triglyceride, there will be two 12-hydroxystearic moieties and one stearic moiety. Alternatively, partial hydrogenation can be used. However, poly(oxyalkylated) castor oils are not suitable because they have unsuitable melting points.

As can be appreciated, commercially supplied glyceride esters such as hydrogenated castor oils can be used including, for example, Thixcin® R. Commercial hydrogenated castor oils are typically supplied in a solid powdered form with each of the particles of the powder being an agglomerate of hydrogenated castor oil. Prior to use in the personal care compositions described herein, it can be useful to deagglomerate and then crystallize particles of the hydrogenated castor oil using shear forces and elevated temperatures. For the personal care compositions described herein, such processing can influence a number of properties of the final personal care composition. For example, processing can increase the number of crystalline particles of the hydrogenated castor oil for a given starting mass of hydrogenated castor oil and can consequently can increase the amount and modify the properties of the coacervate while reducing residue caused by excess hydrogenated castor oil.

A variety of processes are known to process hydrogenated castor oils into suitable crystalline dispersions. For example, a hydrogenated castor oil can be dispersed in oil, mixed at a high shear, and heated to a temperature of about 55 °C to about 60 °C to deagglomerate the particles. Cooling to a temperature below about 35 °C can then form dispersed crystals of the hydrogenated castor oil. As can be appreciated, oil can be a useful dispersion medium because hydrogenated castor oils have limited solubility in aqueous solutions.

Hydrogenated castor oil can be crystallized under aqueous conditions through inclusion of a surfactant. A crystalline premix is formed by combining, under high shear, about 0.30% to about 4%, by weight, of a hydrogenated castor oil, about 15% to about 40%, by weight, of a surfactant, and water. The premix composition can then be heated to a temperature of about 65 °C to about 84 °C and mixed for about 5 minutes to about 20 minutes. Finally, fiber like crystals of hydrogenated castor oil can be formed by cooling the premix composition to about 20 °C by decreasing the temperature about 10 °C/minute to 1 °C/minute under low shear. It is theorized that slow cooling (e.g., about 1.0 °C/minute or less) of the hydrogenated castor oil allows for very thin crystals to form. Low shear during the cooling process can ensure the crystals are not fractured.

As can be appreciated, many variations to this process are possible. For example, a premix composition can be cooled to a temperature of about 20 °C to about 50 °C, and/or to a temperature of from about 25 °C to about 45 °C. However, initial temperatures below about 65 °C or above about 88 °C, produce unsatisfactory crystals due to insufficient deagglomeration of the hydrogenated castor oil or melting of the hydrogenated castor oil respectively. The pH can also be adjusted. For example, the pH can be adjusted to a value of about 5 to about 12, and alternatively to a value of about 6 to about 8.

Any suitable surfactant can be used for an aqueous crystallization process including any of the surfactants suitable for the personal care compositions described herein. The surfactant can be sodium lauryl sulfate and/or sodium laureth sulfate. Additional suitable surfactants, including anionic, amphoteric, cationic, and zwitterionic surfactants, are described in U.S. Patent No. 6,649,155, U.S. Patent Application Publication No. 2008/0317698, and U.S. Patent Application Publication No. 2008/0206355.

As can be appreciated, aqueous crystallization of hydrogenated castor oils can suitable, as such processes can facilitate the inclusion of the hydrogenated castor oil crystals into a personal care composition. For example, aqueous processing of a hydrogenated castor oil can be utilized to form a premix which can subsequently be mixed with additional

components to directly form a personal care composition.

The hydrogenated castor oil can be crystallized into a fiber shape. For example, about 80% to about 100% of the crystals can be in a fiber shape and about 80% to about 100% of the crystals can be about 5 micrometers or longer, alternatively about 80% to about 100% of the fiber shaped crystals can be about 10 micrometers or longer. The crystals can be from about 5, 10, 20, 30 micrometers and/or to about 200, 100, 50, 45, 40 and/or 30 micrometers in length, alternatively the fiber length can be about 10 micrometers to about 40 micrometers in length and the width of the fibers can be about 0.5 micrometer to about 2.0 micrometers. Suitable crystals can have an aspect ratio higher than 5X, alternatively crystals can have an aspect ratio higher than 10X.

Additional processes suitable to crystallize hydrogenated castor oil are disclosed in U.S. Patent No. 9,138,429.

As can be appreciated, the quality of the glyceride ester crystals can have additional effects on the personal care compositions described herein. For example, glyceride ester crystals of excellent quality, such as fiber shaped hydrogenated castor oil crystals, can additionally act as a structurant for the personal care compositions. If including such crystals, a personal care composition can reduce, or eliminate, the need to use any additional structuring or suspending agents. The personal care composition can be substantially free of additional (other than glyceride ester crystals) structuring and suspending agents. As used herein substantially free of additional (other than glyceride ester crystals) structuring and suspending agents is from about 0 to about 0.025 wt. %, alternatively from about 0 to about 0.05 wt.%, alternatively from about 0 to about 0.25 wt. %, and alternatively from about 0 to about 0.5 wt. %. Glyceride ester crystals having poor, or irregular, geometry, in contrast, can still increase the quantity of coacervate and improve the coacervate properties of a personal care composition but can exhibit reduced structuring of the compositions. Such personal care compositions may utilize additional structuring or suspending agents.

A personal care composition can include about 0.01% to about 2%, by weight, of suitable glyceride ester crystals, about 0.01% to about 1.5%, by weight, of suitable glyceride ester crystals, about 0.03% to about 1%, by weight, of suitable glyceride ester crystals, about 0.03% to about .5%, by weight, of suitable glyceride ester crystals about 0.04% to about 0.50%, by weight, of suitable glyceride ester crystals, about 0.04% to about 0.15%, by weight, of suitable glyceride ester crystals, about 0.05% to about 0.08%, by weight, of suitable glyceride ester crystals, about 0.05% to about 0.25%, by weight, of suitable glyceride ester crystals and about 0.06%, by weight, of suitable glyceride ester crystals.

## B. Surfactant

The personal care compositions described herein can include one or more detersive surfactants. As can be appreciated, detersive surfactants provide a cleaning benefit to soiled articles such as hair, skin, and hair follicles by facilitating the removal of oil and other soils. Surfactants generally facilitate such cleaning due to their amphiphilic nature which allows for the surfactants to break up, and form micelles around, oil and other soils which can then be rinsed out, thereby removing them from the soiled article. Suitable detersive surfactants for a personal care composition can include anionic moieties to allow for the formation of a coacervate with a cationic polymer. The detersive surfactant can be selected from anionic surfactants, amphoteric surfactants, and zwitterionic surfactants.

Personal care compositions can also, or alternatively, include a combination of multiple surfactants such as a mixture of amphoteric surfactants and non-ionic surfactants. As can be appreciated, glyceride ester crystals have been discovered to increase the amount of coacervate formed allowing for personal care compositions to include greater quantities of, for example, non-ionic surfactants while still producing suitable quantities of coacervate.

### 1. Anionic Surfactants

Suitable anionic surfactants for personal care compositions described herein can include alkyl and alkyl ether sulfates as well as water-soluble salts of organic, sulfuric acid reaction products. For example, a suitable anionic surfactant can include one or more of ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, and monoethanolamine lauryl sulfate. The surfactant can be chosen from one or more of sodium lauryl sulfate and sodium laureth sulfate.

A personal care composition can alternatively be substantially free of sulfate surfactants. As used here in, substantially free of sulfate surfactants means from about 0 to about 0.1% or less, alternatively from about 0 to about 0.05%, alternatively from about 0 to about 0.01%, and alternatively 0%. As can be appreciated, such compositions can have greater consumer acceptance. Suitable anionic surfactants can alternatively include isethionate, sarcosinate, sulfonate, sulfosuccinate, sulfoacetate, glycinate, glutamate, glucosecarboxylate, and phosphate ester surfactants.

Suitable anionic surfactants can include water-soluble olefin sulfonates which have the general formula $R^1$-$SO_3M$ where $R^1$ is a straight or branched chain, saturated, aliphatic hydrocarbon radical having from 10 to 24 carbon atoms, 10 to 18 carbon atoms, or from 13 to 15 carbon atoms; and M is a water soluble cation such as ammonium, sodium, potassium, triethanolamine cation, or salts of the divalent magnesium ion with two anionic surfactant anions. Suitable olefin sulfonates such as sodium paraffin sulfonates can be produced through the reaction of $SO_2$ and $O_2$ with a suitable chain length paraffin.

Suitable olefin sulfonates can also contain minor amounts of other materials, such as alkene disulfonates depending upon the reaction conditions, proportion of reactants, the nature of the starting olefins and impurities in the olefin stock and side reactions during the sulfonation process. Examples of additional olefin sulfonates and mixtures thereof are described in U.S. Patent No. 3,332,880.

Another class of suitable sulfate-free anionic detersive surfactants includes the beta-alkyloxy alkane sulfonates. Beta-alkyloxy alkane sulfonates surfactants conform to Formula I:

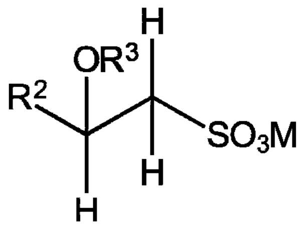

Formula I

where $R^2$ is a straight chain alkyl group having from 6 to 20 carbon atoms, $R^3$ is a lower alkyl group having from 1 to 3 carbon atoms, alternatively 1 carbon atom, and M is a water-soluble cation as previously described in the water-soluble olefin sulfonates.

Suitable sulfate-free anionic detersive surfactants can include isethionate surfactants. For example, suitable isethionate surfactants can include the reaction product of fatty acids esterified with isethionic acid and neutralized with sodium hydroxide. Suitable fatty acids for isethionate surfactants can be derived from coconut oil or palm kernel oil including amides of methyl tauride. Additional examples of suitable isethionic anionic surfactants are described in U.S. Pat. No. 2,486,921; U.S. Pat. No. 2,486,922; and U.S. Pat. No. 2,396,278.

Suitable detersive anionic surfactants can be succinate surfactants. Examples of suitable succinate surfactants can include disodium N-octadecylsulfo succinnate, disodium lauryl sulfosuccinate, diammonium lauryl sulfosuccinate, tetrasodium N-(1,2-dicarboxyethyl)-N-octadecylsulfosuccinnate, diamyl ester of sodium sulfosuccinic acid, dihexyl ester of sodium sulfosuccinic acid, and dioctyl esters of sodium sulfosuccinic acid.

Suitable sulfate-free anionic detersive surfactants can include one or more of sodium cocoyl isethionate ("SCI"), sodium lauroyl methyl isethionate ("SLMI"), sodium lauroyl sarcosinate, sodium $C_{12}$-$C_{14}$ olefin sulfonate, sodium lauroyl glycinate, sodium cocoamphoacetate, sodium cocoyl glutamate, sodium lauryl glucosecarboxylate, sodium lauryl sulfosuccinate, sodium laureth sulfosuccinate, sodium lauryl sulfoacetate, lauryl sarcosine, cocoyl sarcosine, sodium methyl lauroyl taurate, sodium methyl lauroyl taurate, sodium tridecyl benzene sulfonate, sodium dodecyl benzene sulfonate, phosphate ester surfactants, and fatty acid surfactants.

2. Amphoteric Surfactants

A personal care composition can include a suitable amphoteric detersive surfactant. Generally any amphoteric surfactant known for use in hair care or other personal care compositions can be suitable. For example, amphoteric detersive surfactants suitable for inclusion in a personal care composition can include those surfactants broadly described as derivatives of aliphatic secondary and tertiary amines in which the aliphatic radical can be straight or branched chain and wherein one of the aliphatic substituents contains from 8 to 18 carbon atoms and one aliphatic substituent contains an anionic group such as a carboxy, sulfate, sulfonate, phosphate, or phosphonate group. Suitable amphoteric detersive surfactants can include cocoamphoacetate, cocoamphodiacetate, lauroamphoacetate, lauroamphodiacetate, and mixtures thereof. Other suitable amphoteric surfactants include amidobetaines and amidosulfobetaines.

3. Zwitterionic Surfactants

A personal care composition can include a suitable zwitterionic detersive surfactant. For example, a personal care composition can include surfactants broadly described as derivatives of aliphatic quaternary ammonium, phosphonium, and sulfonium compounds, in which the aliphatic radicals can be straight or branched chain, and wherein one of the aliphatic substituents contains from 8 to 18 carbon atoms and one aliphatic substituent contains an anionic group such as carboxy, sulfonate, phosphate or phosphonate group. Betaine zwitterionic surfactants, including high alkyl betaines, can

be beneficial.

Examples of betaine zwitterionic surfactants can include coco dimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, lauryl bis-(2-hydroxypropyl)alpha-carboxyethyl betaine, and mixtures thereof. Examples of sulfobetaines can include coco dimethyl sulfopropyl betaine, stearyl dimethyl sulfopropyl betaine, lauryl dimethyl sulfoethyl betaine, lauryl bis-(2-hydroxyethyl) sulfopropyl betaine and mixtures thereof.

4. Non-Ionic Surfactants

A personal care composition can include a non-ionic detersive surfactant. Generally, suitable non-ionic surfactants can include compounds produced by the condensation of alkylene oxide groups (hydrophilic in nature) with an organic hydrophobic compound, which may be aliphatic or alkyl aromatic in nature. Examples of suitable non-ionic detersive surfactants can include:

1. The polyethylene oxide condensates of alkyl phenols. For example, the condensation products of alkyl phenols having an alkyl group containing from 6 to 20 carbon atoms in either a straight chain or branched chain configuration, with ethylene oxide, the ethylene oxide being present in amounts equal to from about 10 to about 60 moles of ethylene oxide per mole of alkyl phenol.
2. Those derived from the condensation of ethylene oxide with the product resulting from the reaction of propylene oxide and ethylene diamine products.
3. The condensation product of aliphatic alcohols having from 8 to 18 carbon atoms, in either straight chain or branched chain configuration, with ethylene oxide, e.g., a coconut alcohol ethylene oxide condensate having from about 10 to about 30 moles of ethylene oxide per mole of coconut alcohol, the coconut alcohol fraction having from 10 to 14 carbon atoms.
4. Long chain tertiary amine oxides corresponding to the following general formula:

$$R^8R^9R^{10}N \rightarrow O$$

wherein $R^8$ contains an alkyl, alkenyl or monohydroxy alkyl radical of from 8 to 18 carbon atoms, from 0 to about 10 ethylene oxide moieties, and from 0 to about 1 glyceryl moieties, and $R^9$ and $R^{10}$ contain from 1 to 3 carbon atoms and from 0 to about 1 hydroxy groups, e.g., methyl, ethyl, propyl, hydroxyethyl, or hydroxypropyl radicals. The arrow in the formula is a conventional representation of a semipolar bond.
5. Long chain tertiary phosphine oxides corresponding to the following general formula:

$$R^{11}R^{12}R^{13}P \rightarrow O$$

wherein $R^{11}$ contains an alkyl, alkenyl or monohydroxyalkyl radical ranging from 8 to 18 carbon atoms in chain length, from 0 to about 10 ethylene oxide moieties and from 0 to about 1 glyceryl moieties and $R^{12}$ and $R^{13}$ are each alkyl or monohydroxyalkyl groups containing from 1 to 3 carbon atoms.
6. Long chain dialkyl sulfoxides containing one short chain alkyl or hydroxy alkyl radical of from 1 to 3 carbon atoms (usually methyl) and one long hydrophobic chain which include alkyl, alkenyl, hydroxy alkyl, or keto alkyl radicals containing from 8 to 20 carbon atoms, from 0 to about 10 ethylene oxide moieties and from 0 to about 1 glyceryl moiety.
7. Alkyl polysaccharide ("APS") surfactants such as the alkyl polyglycosides. Such surfactants are described in U.S. Patent No. 4,565,647. APS surfactants can include a hydrophobic group with 6 to 30 carbon atoms and can include polysaccharide (e.g., polyglycoside) as the hydrophilic group. Optionally, there can be a polyalkylene-oxide group joining the hydrophobic and hydrophilic moieties. The alkyl group (i.e., the hydrophobic moiety) can be saturated or unsaturated, branched or unbranched, and unsubstituted or substituted (e.g., with hydroxy or cyclic rings).
8. Polyethylene glycol (PEG) glyceryl fatty esters, such as those of the formula $R(O)OCH_2CH(OH)CH_2(OCH_2CH_2)_nOH$ wherein n is from 5 to 200 or from 20 to 100, and R is an aliphatic hydrocarbyl having from 8 to 20 carbon atoms.
9. Glucoside surfactants including, for example, lauryl glucoside, coco glucoside, and decyl glucoside.
10. Certain surfactant-emulsifying compounds such as laureth-4.

Examples of non-ionic detersive surfactants suitable for inclusion in a personal care composition can include cocamide, cocamide methyl MEA, cocamide DEA, cocamide MEA, cocamide MIPA, lauramide DEA, lauramide MEA, lauramide MIPA, myristamide DEA, myristamide MEA, PEG-20 cocamide MEA, PEG-2 cocamide, PEG-3 cocamide,

PEG-4 cocamide, PEG-5 cocamide, PEG-6 cocamide, PEG-7 cocamide, PEG-3 lauramide, PEG-5 lauramide, PEG-3 oleamide, PPG-2 cocamide, PPG-2 hydroxyethyl cocamide, and mixtures thereof.

Additional examples and descriptions of suitable detersive surfactants are set forth in McCutcheon's, Emulsifiers and Detergents, 1989 Annual, published by M. C. Publishing Co., U.S. Patent No. 2,438,091, U.S. Patent No. 2,528,378, U.S. Patent No. 2,658,072, U.S. Patent No. 3,929,678, U.S. Patent No. 5,104,646, and U.S. Patent No. 5,106,609, U.S. Patent No. 6,649,155; U.S. Patent Application Publication No. 2008/0317698; and U.S. Patent Application Publication No. 2008/0206355.

The concentration of a detersive surfactant in personal care compositions described herein can be selected based on the desired cleaning and lather performance of the personal care composition. The amount of detersive surfactant can be about 2% to about 50%, by weight; alternatively, from about 5% to about 30%, by weight; alternatively, from about 8% to about 25%, by weight; alternatively, from about 10% to about 20%, by weight; alternatively, about 5%, by weight; alternatively, about 10%, by weight; alternatively, about 12%, by weight; alternatively, about 15%, by weight; alternatively, about 17%, by weight; alternatively, about 18%, by weight; and alternatively, about 20%, by weight.

## C. Cationic Polymer

A personal care composition can include a cationic polymer to allow formation of a coacervate. As can be appreciated, the cationic charge of a cationic polymer can interact with an anionic charge of a surfactant to form the coacervate. Suitable cationic polymers can include: (a) a cationic guar polymer, (b) a cationic non-guar galactomannan polymer, (c) a cationic starch polymer, (d) a cationic copolymer of acrylamide monomers and cationic monomers, (e) a synthetic, non-crosslinked, cationic polymer, which may or may not form lyotropic liquid crystals upon combination with the detersive surfactant, and (f) a cationic cellulose polymer. In certain examples, more than one cationic polymer can be included.

Personal care compositions described herein having a single cationic polymer, rather than a blend of more than one cationic polymers, can provide good hair feel. For example, personal care compositions including a single cationic guar polymer and crystallized trihydroxystearin (a hydrogenated castor oil) provide improved lather creaminess and improved hair wet feel compared to the compositions without the trihydroxystearin. Composition including the trihydroxystearin also allow hair to detangle when wet with greater ease than compositions without the trihydroxystearin.

The inclusion of crystallized trihydroxystearin also results in a personal care compositions with a greater quantity of coacervate than similar compositions without crystallized trihydroxystearin.

As can be appreciated, personal care compositions including only a single cationic polymer can have numerous benefits. For example, personal care compositions including only a single cationic polymer can be formulated into compositions having a desired light transmittance more easily due to smaller polymer loading levels. For example, personal care compositions including a coacervate can be transparent. Additionally, such personal care compositions are easier to manufacture and can be easier to optimize for multiple properties. The use of trihydroxystearin can also allow personal care compositions to include other classes of cationic polymer including, for example, cassia and tapioca polymers.

A cationic polymer can be included by weight of the personal care composition at about 0.05% to about 3%, about 0.075% to about 2.0%, or at about 0.1% to about 1.0%. Cationic polymers can have cationic charge densities of about 0.9 meq/g or more, about 1.2 meq/g or more, and about 1.5 meq/g or more. However, cationic charge density can also be about 7 meq/g or less and alternatively about 5 meq/g or less. The charge densities can be measured at the pH of intended use of the personal care composition. (e.g., at about pH 3 to about pH 9; or about pH 4 to about pH 8). The average molecular weight of cationic polymers can generally be between about 10,000 and 10 million, between about 50,000 and about 5 million, and between about 100,000 and about 3 million, and between about 100,000 and about 2.5 million. Low molecular weight cationic polymers can be used. Low molecular weight cationic polymers can have greater translucency in the liquid carrier of a personal care composition. The cationic polymer can be a single type, such as the cationic guar polymer guar hydroxypropyltrimonium chloride having a weight average molecular weight of about 2.5 million g/mol or less, and the personal care composition can be substantially free of additional cationic polymers. As used herein, substantially free of additional cationic polymers means from about 0 to about 0.05 of an additional cationic polymer.

### Cationic Guar Polymer

The cationic polymer can be a cationic guar polymer, which is a cationically substituted galactomannan (guar) gum derivative. Suitable guar gums for guar gum derivatives can be obtained as a naturally occurring material from the seeds of the guar plant. As can be appreciated, the guar molecule is a straight chain mannan which is branched at regular intervals with single membered galactose units on alternative mannose units. The mannose units are linked to each other by means of $\beta$(1-4) glycosidic linkages. The galactose branching arises by way of an $\alpha$(1-6) linkage. Cationic derivatives of the guar gums can be obtained through reactions between the hydroxyl groups of the polygalactomannan and reactive

quaternary ammonium compounds. The degree of substitution of the cationic groups onto the guar structure can be sufficient to provide the requisite cationic charge density described above.

A cationic guar polymer can have a weight average molecular weight ("M.Wt.") of less than about 2.5 million g/mol, and can have a charge density from about 0.05 meq/g to about 2.5 meq/g. Alternatively, the cationic guar polymer can have a weight average M.Wt. of less than 1.5 million g/mol, from about 150 thousand g/mol to about 1.5 million g/mol, from about 200 thousand g/mol to about 1.5 million g/mol, from about 300 thousand g/mol to about 1.5 million g/mol, and from about 700,000 thousand g/mol to about 1.5 million g/mol. The cationic guar polymer can have a charge density from about 0.2 meq/g to about 2.2 meq/g, from about 0.3 meq/g to about 2.0 meq/g, from about 0.4 meq/g to about 1.8 meq/g; and from about 0.5 meq/g to about 1.7 meq/g.

A cationic guar polymer can have a weight average M.Wt. of less than about 1 million g/mol, and can have a charge density from about 0.1 meq/g to about 2.5 meq/g. A cationic guar polymer can have a weight average M.Wt. of less than 900 thousand g/mol, from about 150 thousand to about 800 thousand g/mol, from about 200 thousand g/mol to about 700 thousand g/mol, from about 300 thousand to about 700 thousand g/mol, from about 400 thousand to about 600 thousand g/mol, from about 150 thousand g/mol to about 800 thousand g/mol, from about 200 thousand g/mol to about 700 thousand g/mol, from about 300 thousand g/mol to about 700 thousand g/mol, and from about 400 thousand g/mol to about 600 thousand g/mol. A cationic guar polymer has a charge density from about 0.2 meq/g to about 2.2 meq/g, from about 0.3 meq/g to about 2.0 meq/g, from about 0.4 meq/g to about 1.8 meq/g; and from about 0.5 meq/g to about 1.5 meq/g.

A personal care composition can include from about 0.01% to less than about 0.7%, by weight of the personal care composition of a cationic guar polymer, from about 0.04% to about 0.55%, by weight, from about 0.08% to about 0.5%, by weight, from about 0.16% to about 0.5%, by weight, from about 0.2% to about 0.5%, by weight, from about 0.3% to about 0.5%, by weight, and from about 0.4% to about 0.5%, by weight.

The cationic guar polymer can be formed from quaternary ammonium compounds which conform to general Formula II:

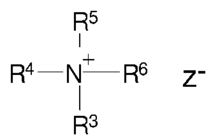

Formula II

wherein where $R^3$, $R^4$ and $R^5$ are methyl or ethyl groups; and $R^6$ is either an epoxyalkyl group of the general Formula III:

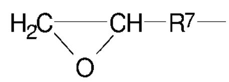

Formula III

or $R^6$ is a halohydrin group of the general Formula IV:

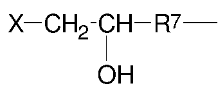

Formula IV

wherein $R^7$ is a $C_1$ to $C_3$ alkylene; X is chlorine or bromine, and Z is an anion such as Cl-, Br-, I- or $HSO_4$-.

Suitable cationic guar polymers can conform to the general formula V:

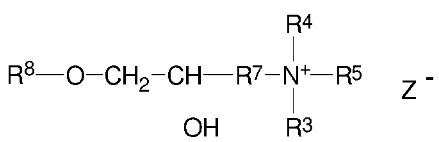

Formula V

wherein $R^8$ is guar gum; and wherein $R^4$, $R^5$, $R^6$ and $R^7$ are as defined above; and wherein Z is a halogen. Suitable cationic guar polymers can conform to Formula VI:

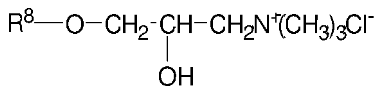

Formula VI

wherein $R^8$ is guar gum.

Suitable cationic guar polymers can also include cationic guar gum derivatives, such as guar hydroxypropyltrimonium chloride. Suitable examples of guar hydroxypropyltrimonium chlorides can include the Jaguar® series commercially available from Solvay S.A., Hi-Care Series from Rhodia, and N-Hance and AquaCat from Ashland Inc. Jaguar® C-500 has a charge density of 0.8 meq/g and a M.Wt. of 500,000 g/mole; Jaguar® C-17 has a cationic charge density of about 0.6 meq/g and a M.Wt. of about 2.2 million g/mol; Jaguar® C 13S has a M.Wt. of 2.2 million g/mol and a cationic charge density of about 0.8 meq/g; Hi-Care 1000 has a charge density of about 0.7 meq/g and a M.Wt. of about 600,000 g/mole; N-Hance 3269 and N-Hance 3270, have a charge density of about 0.7 meq/g and a M.Wt. of about 425,000 g/mole; N-Hance 3196 has a charge density of about 0.8 meq/g and a M.Wt. of about 1,100,000 g/ mole; and AquaCat CG518 has a charge density of about 0.9 meq/g and a M.Wt. of about 50,000 g/mole. N-Hance BF-13 and N-Hance BF-17 are borate (boron) free guar polymers. N-Hance BF-13 has a charge density of about 1.1 meq/g and M.W.t of about 800,000 and N-Hance BF-17 has a charge density of about 1.7 meq/g and M.W.t of about 800,000.

Cationic Non-Guar Galactomannan Polymer

The cationic polymer can be a galactomannan polymer derivative. Suitable galactomannan polymer can have a mannose to galactose ratio of greater than 2:1 on a monomer to monomer basis and can be a cationic galactomannan polymer derivative or an amphoteric galactomannan polymer derivative having a net positive charge. As used herein, the term "cationic galactomannan" refers to a galactomannan polymer to which a cationic group is added. The term "amphoteric galactomannan" refers to a galactomannan polymer to which a cationic group and an anionic group are added such that the polymer has a net positive charge.

Galactomannan polymers can be present in the endosperm of seeds of the Leguminosae family. Galactomannan polymers are made up of a combination of mannose monomers and galactose monomers. The galactomannan molecule is a straight chain mannan branched at regular intervals with single membered galactose units on specific mannose units. The mannose units are linked to each other by means of β (1-4) glycosidic linkages. The galactose branching arises by way of an α (1-6) linkage. The ratio of mannose monomers to galactose monomers varies according to the species of the plant and can be affected by climate. Non Guar Galactomannan polymer derivatives can have a ratio of mannose to galactose of greater than 2:1 on a monomer to monomer basis. Suitable ratios of mannose to galactose can also be greater than 3:1 or greater than 4:1. Analysis of mannose to galactose ratios is well known in the art and is typically based on the measurement of the galactose content.

The gum for use in preparing the non-guar galactomannan polymer derivatives can be obtained from naturally occurring materials such as seeds or beans from plants. Examples of various non-guar galactomannan polymers include Tara gum (3 parts mannose/1 part galactose), Locust bean or Carob (4 parts mannose/1 part galactose), and Cassia gum (5 parts mannose/1 part galactose).

A non-guar galactomannan polymer derivative can have a M. Wt. from about 1,000 g/mol to about 10,000,000 g/mol, and a M.Wt. from about 5,000 g/mol to about 3,000,000 g/mol.

The personal care compositions described herein can include galactomannan polymer derivatives which have a cationic charge density from about 0.5 meq/g to about 7 meq/g. The galactomannan polymer derivatives can have a cationic charge density from about 1 meq/g to about 5 meq/g. The degree of substitution of the cationic groups onto the galactomannan structure can be sufficient to provide the requisite cationic charge density.

A galactomannan polymer derivative can be a cationic derivative of the non-guar galactomannan polymer, which is obtained by reaction between the hydroxyl groups of the polygalactomannan polymer and reactive quaternary ammonium compounds. Suitable quaternary ammonium compounds for use in forming the cationic galactomannan polymer derivatives include those conforming to the general Formulas II to VI, as defined above.

Cationic non-guar galactomannan polymer derivatives formed from the reagents described above can be represented by the general Formula VII:

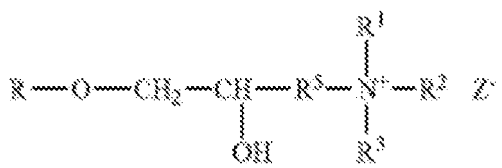

Formula VII

wherein R is the gum. The cationic galactomannan derivative can be a gum hydroxypropyltrimethylammonium chloride, which can be more specifically represented by the general Formula VIII:

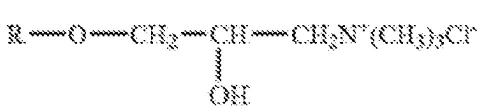

Formula VIII

The galactomannan polymer derivative can be an amphoteric galactomannan polymer derivative having a net positive charge, obtained when the cationic galactomannan polymer derivative further comprises an anionic group.

A cationic non-guar galactomannan can have a ratio of mannose to galactose which is greater than about 4:1, a M.Wt. of about 100,000 g/mol to about 500,000 g/mol, a M.Wt. of about 50,000 g/mol to about 400,000 g/mol, and a cationic charge density from about 1 meq/g to about 5 meq/g, and from about 2 meq/ g to about 4 meq/g.

Personal care compositions can include at least about 0.05% of a galactomannan polymer derivative by weight of the composition. The personal care compositions can include from about 0.05% to about 2%, by weight of the composition, of a galactomannan polymer derivative.

Cationic Starch Polymers

Suitable cationic polymers can also be water-soluble cationically modified starch polymers. As used herein, the term "cationically modified starch" refers to a starch to which a cationic group is added prior to degradation of the starch to a smaller molecular weight, or wherein a cationic group is added after modification of the starch to achieve a desired molecular weight. The definition of the term "cationically modified starch" also includes amphoterically modified starch. The term "amphoterically modified starch" refers to a starch hydrolysate to which a cationic group and an anionic group are added.

The personal care compositions described herein can include cationically modified starch polymers at a range of about 0.01% to about 10%, and/or from about 0.05% to about 5%, by weight of the composition.

The cationically modified starch polymers disclosed herein have a percent of bound nitrogen of from about 0.5% to about 4%.

The cationically modified starch polymers can have a molecular weight from about 850,000 g/mol to about 15,000,000 g/mol and from about 900,000 g/mol to about 5,000,000 g/mol.

Cationically modified starch polymers can have a charge density of from about 0.2 meq/g to about 5 meq/g, and from about 0.2 meq/g to about 2 meq/g. The chemical modification to obtain such a charge density can include the addition of amino and/or ammonium groups into the starch molecules. Non-limiting examples of such ammonium groups can include substituents such as hydroxypropyl trimmonium chloride, trimethylhydroxypropyl ammonium chloride, dimethylstearylhydroxypropyl ammonium chloride, and dimethyldodecylhydroxypropyl ammonium chloride. Further details are described in Solarek, D. B., Cationic Starches in Modified Starches: Properties and Uses, Wurzburg, O. B., Ed., CRC Press, Inc., Boca Raton, Fla. 1986, pp 113-125, The cationic groups can be added to the starch prior to degradation to a smaller molecular weight or the cationic groups may be added after such modification.

A cationically modified starch polymer can have a degree of substitution of a cationic group from about 0.2 to about 2.5. As used herein, the "degree of substitution" of the cationically modified starch polymers is an average measure of the number of hydroxyl groups on each anhydroglucose unit which is derivatized by substituent groups. Since each anhydroglucose unit has three potential hydroxyl groups available for substitution, the maximum possible degree of substitution is 3. The degree of substitution is expressed as the number of moles of substituent groups per mole of anhydroglucose unit, on a molar average basis. The degree of substitution can be determined using proton nuclear magnetic resonance spectroscopy ("$^1$H NMR") methods well known in the art. Suitable $^1$H NMR techniques include those described in "Observation on NMR Spectra of Starches in Dimethyl Sulfoxide, Iodine-Complexing, and Solvating in Water-Dimethyl Sulfoxide", Qin-Ji Peng and Arthur S. Perlin, Carbohydrate Research, 160 (1987), 57-72; and "An Approach to the Structural Analysis of Oligosaccharides by NMR Spectroscopy", J. Howard Bradbury and J. Grant Collins, Carbohydrate Research, 71, (1979), 15-25.

The source of starch before chemical modification can be selected from a variety of sources such as tubers, legumes, cereal, and grains. For example, starch sources can include corn starch, wheat starch, rice starch, waxy corn starch, oat starch, cassaya starch, waxy barley, waxy rice starch, glutenous rice starch, sweet rice starch, amioca, potato starch, tapioca starch, oat starch, sago starch, sweet rice, or mixtures thereof. Suitable cationically modified starch polymers can be selected from degraded cationic maize starch, cationic tapioca, cationic potato starch, and mixtures thereof. Cationically modified starch polymers are cationic corn starch and cationic tapioca.

The starch, prior to degradation or after modification to a smaller molecular weight, can include one or more additional modifications. For example, these modifications may include crosslinking, stabilization reactions, phosphorylations, and hydrolyzations. Stabilization reactions can include alkylation and esterification.

Cationically modified starch polymers can be included in a personal care composition in the form of hydrolyzed starch (e.g., acid, enzyme, or alkaline degradation), oxidized starch (e.g., peroxide, peracid, hypochlorite, alkaline, or any other oxidizing agent), physically/mechanically degraded starch (e.g., via the thermo-mechanical energy input of the processing equipment), or combinations thereof.

The starch can be readily soluble in water and can form a substantially translucent solution in water. The transparency of the composition is measured by Ultra-Violet/Visible ("UV/VIS") spectrophotometry, which determines the absorption or transmission of UV/VIS light by a sample, using a Gretag Macbeth Colorimeter Color. A light wavelength of

600 nm has been shown to be adequate for characterizing the degree of clarity of personal care compositions.

Cationic Copolymer of an Acrylamide Monomer and a Cationic Monomer

A personal care composition can include a cationic copolymer of an acrylamide monomer and a cationic monomer, wherein the copolymer has a charge density of from about 1.0 meq/g to about 3.0 meq/g. The cationic copolymer can be a synthetic cationic copolymer of acrylamide monomers and cationic monomers.

Suitable cationic polymers can include:

(i) an acrylamide monomer of the following Formula IX:

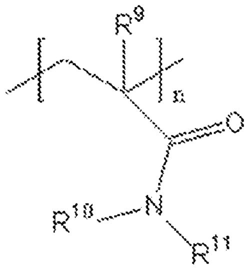

Formula IX

where $R^9$ is H or $C_{1-4}$ alkyl; and $R^{10}$ and $R^{11}$ are independently selected from the group consisting of H, $C_{1-4}$ alkyl, $CH_2OCH_3$, $CH_2OCH_2CH(CH_3)_2$, and phenyl, or together are $C_{3-6}$cycloalkyl; and (ii) a cationic monomer conforming to Formula X:

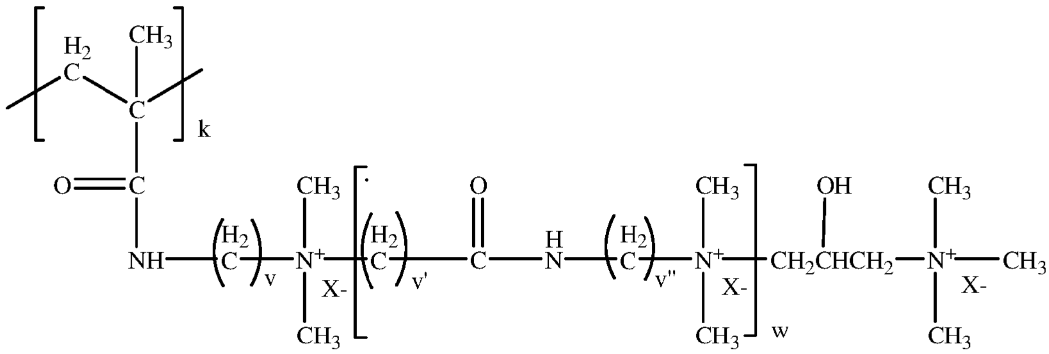

Formula X

where k = 1, each of v, v', and v" is independently an integer of from 1 to 6, w is zero or an integer of from 1 to 10, and $X^-$ is an anion.

A cationic monomer can conform to Formula X where k = 1, v = 3 and w = 0, z = 1 and $X^-$ is $Cl^-$ to form the following structure (Formula XI):

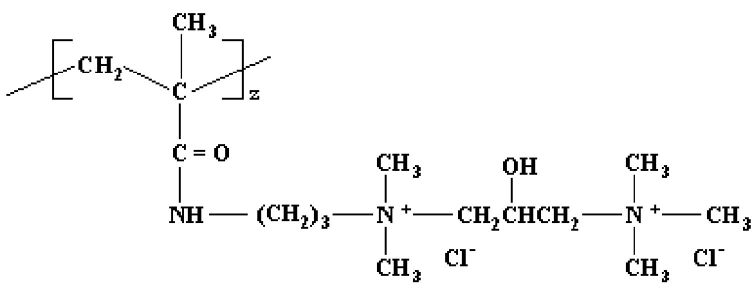

Formula XI

As can be appreciated, the above structure can be referred to as diquat.

A cationic monomer can conform to Formula X wherein v and v" are each 3, v' = 1, w =1, y = 1 and $X^-$ is $Cl^-$, to form

the following structure of Formula XII:

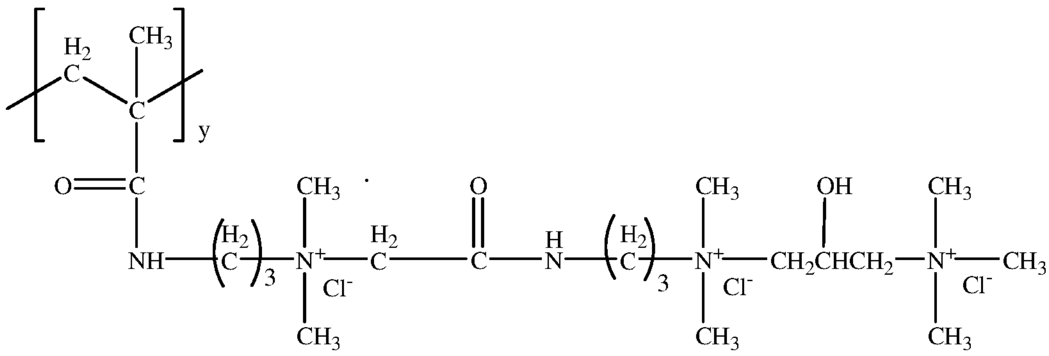

Formula XII

The structure of Formula XII can be referred to as triquat.

The acrylamide monomer can be either acrylamide or methacrylamide.

The cationic copolymer can be AM:TRIQUAT which is a copolymer of acrylamide and 1,3-Propanediaminium,N-[2-[[[dimethyl[3-[(2-methyl-1-oxo-2-propenyl)amino]propyl]ammonio]acetyl]amino]ethyl]2-hydroxy-N,N,N',N',N'-pentamethyl-, trichloride. AM:TRIQUAT is also known as polyquaternium 76 (PQ76). AM:TRIQUAT can have a charge density of 1.6 meq/g and a M.Wt. of 1.1 million g/mol.

The cationic copolymer can include an acrylamide monomer and a cationic monomer, wherein the cationic monomer is selected from the group consisting of: dimethylaminoethyl (meth)acrylate, dimethylaminopropyl (meth) acrylate, ditertiobutylaminoethyl (meth)acrylate, dimethylaminomethyl (meth)acrylamide, dimethylaminopropyl (meth) acrylamide; ethylenimine, vinylamine, 2-vinylpyridine, 4- vinylpyridine; trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride, and mixtures thereof.

The cationic copolymer can include a cationic monomer selected from the group consisting of: trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, and mixtures thereof.

The cationic copolymer can be formed from (1) copolymers of (meth)acrylamide and cationic monomers based on (meth)acrylamide, and/or hydrolysis-stable cationic monomers, (2) terpolymers of (meth)acrylamide, monomers based on cationic (meth)acrylic acid esters, and monomers based on (meth)acrylamide, and/or hydrolysis-stable cationic monomers. Monomers based on cationic (meth)acrylic acid esters can be cationized esters of the (meth)acrylic acid containing a quaternized N atom. Cationized esters of the (meth)acrylic acid containing a quaternized N atom can be quaternized dialkylaminoalkyl (meth)acrylates with $C_1$ to $C_3$ in the alkyl and alkylene groups. The cationized esters of the (meth)acrylic acid containing a quaternized N atom can be selected from the group consisting of: ammonium salts of dimethylaminomethyl (meth)acrylate, dimethylaminoethyl (meth)acrylate, dimethylaminopropyl (meth)acrylate, diethylaminomethyl (meth)acrylate, diethylaminoethyl (meth)acrylate; and diethylaminopropyl (meth)acrylate quaternized with methyl chloride. The cationized esters of the (meth)acrylic acid containing a quaternized N atom can be dimethylaminoethyl acrylate, which is quaternized with an alkyl halide, or with methyl chloride or benzyl chloride or dimethyl sulfate (ADAME-Quat). The cationic monomer when based on (meth)acrylamides are quaternized dialkylaminoalkyl(meth) acrylamides with $C_1$ to $C_3$ in the alkyl and alkylene groups, or dimethylaminopropylacrylamide, which is quaternized with an alkyl halide, or methyl chloride or benzyl chloride or dimethyl sulfate.

The cationic monomer based on a (meth)acrylamide can be a quaternized dialkylaminoalkyl(meth)acrylamide with $C_1$ to $C_3$ in the alkyl and alkylene groups. The cationic monomer based on a (meth)acrylamide can be dimethylaminopropylacrylamide, which is quaternized with an alkyl halide, especially methyl chloride or benzyl chloride or dimethyl sulfate.

The cationic monomer can be a hydrolysis-stable cationic monomer. Hydrolysis-stable cationic monomers can be, in addition to a dialkylaminoalkyl(meth)acrylamide, any monomer that can be regarded as stable to the OECD hydrolysis test. The cationic monomer can be hydrolysis-stable and the hydrolysis-stable cationic monomer can be selected from the group consisting of: diallyldimethylammonium chloride and water-soluble, cationic styrene derivatives.

The cationic copolymer can be a terpolymer of acrylamide, 2-dimethylammoniumethyl (meth)acrylate quaternized with methyl chloride (ADAME-Q) and 3-dimethylammoniumpropyl(meth)acrylamide quaternized with methyl chloride (DIMAPA-Q). The cationic copolymer can be formed from acrylamide and acrylamidopropyltrimethylammonium chloride, wherein the acrylamidopropyltrimethylammonium chloride has a charge density of from about 1.0 meq/g to about 3.0 meq/g.

The cationic copolymer can have a charge density of from about 1.1 meq/g to about 2.5 meq/g, from about 1.1 meq/g to about 2.3 meq/g, from about 1.2 meq/g to about 2.2 meq/g, from about 1.2 meq/g to about 2.1 meq/g, from about 1.3 meq/g to about 2.0 meq/g, and from about 1.3 meq/g to about 1.9 meq/g.

The cationic copolymer can have a M.Wt. from about 100 thousand g/mol to about 2 million g/mol, from about 300 thousand g/mol to about 1.8 million g/mol, from about 500 thousand g/mol to about 1.6 million g/mol, from about 700 thousand g/mol to about 1.4 million g/mol, and from about 900 thousand g/mol to about 1.2 million g/mol.

The cationic copolymer can be a trimethylammoniopropylmethacrylamide chloride-N-Acrylamide copolymer, which is also known as AM:MAPTAC. AM:MAPTAC can have a charge density of about 1.3 meq/g and a M.Wt. of about 1.1 million g/mol. The cationic copolymer can be AM:ATPAC. AM:ATPAC can have a charge density of about 1.8 meq/g and a M.Wt. of about 1.1 million g/mol.

<u>Synthetic Polymers</u>

A cationic polymer can be a synthetic polymer that is formed from:

i) one or more cationic monomer units, and optionally
ii) one or more monomer units bearing a negative charge, and/or
iii) a nonionic monomer,

wherein the subsequent charge of the copolymer is positive. The ratio of the three types of monomers is given by "m", "p" and "q" where "m" is the number of cationic monomers, "p" is the number of monomers bearing a negative charge and "q" is the number of nonionic monomers.

The cationic polymers can be water soluble or dispersible, non-crosslinked, and synthetic cationic polymers which have the structure of Formula XIII:

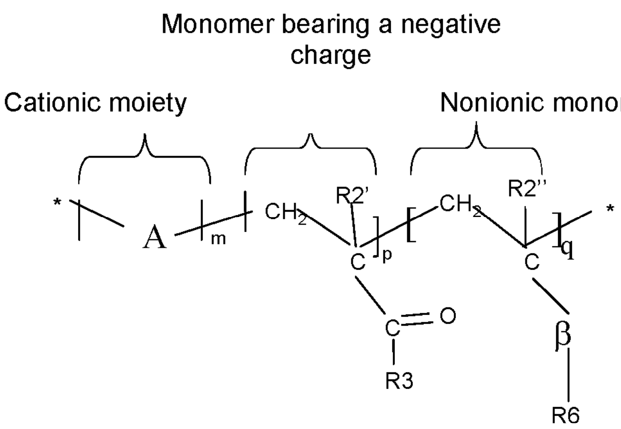


Formula XIII

$m \geq 1$
$p = 0$ or $\geq 1$
$q = 0$ or $\geq 1$
$m \geq p$

where A, may be one or more of the following cationic moieties:

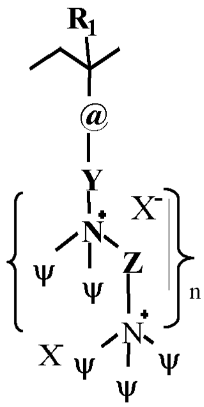 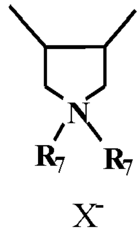 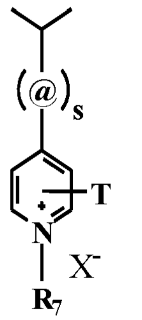

where @ = amido, alkylamido, ester, ether, alkyl or alkylaryl;
where Y = C1-C22 alkyl, alkoxy, alkylidene, alkyl or aryloxy;
where ψ = C1-C22 alkyl, alkyloxy, alkyl aryl or alkyl arylox;.
where Z = C1-C22 alkyl, alkyloxy, aryl or aryloxy;
where R1 = H, C1-C4 linear or branched alkyl;
where s = 0 or 1, n = 0 or ≥ 1;
where T and R7 = C1-C22 alkyl; and
where X- = halogen, hydroxide, alkoxide, sulfate or alkylsulfate.

Where the monomer bearing a negative charge is defined by R2' = H, $C_1$-$C_4$ linear or branched alkyl and R3 is:

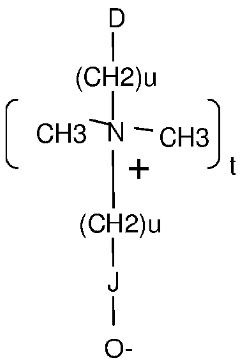 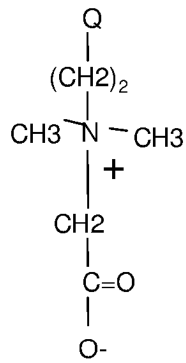 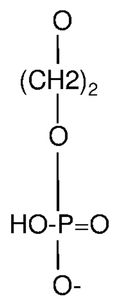 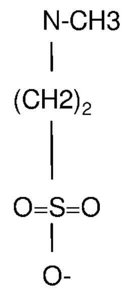

where D = O, N, or S;
where Q = $NH_2$ or O;
where u = 1-6;
where t = 0-1; and
where J = oxygenated functional group containing the following elements P, S, C.

Where the nonionic monomer is defined by R2" = H, $C_1$-$C_4$ linear or branched alkyl, R6 = linear or branched alkyl, alkyl aryl, aryl oxy, alkyloxy, alkylaryl oxy and β is defined as

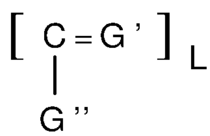
;

and
where G' and G'' are, independently of one another, O, S or N-H and L =0 or 1.

Suitable monomers can include aminoalkyl (meth)acrylates, (meth)aminoalkyl (meth)acrylamides; monomers comprising at least one secondary, tertiary or quaternary amine function, or a heterocyclic group containing a nitrogen atom, vinylamine or ethylenimine; diallyldialkyl ammonium salts; their mixtures, their salts, and macromonomers deriving

from therefrom.

Further examples of suitable cationic monomers can include dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide, ethylenimine, vinylamine, 2-vinylpyridine, 4- vinylpyridine, trimethylammonium ethyl (meth) acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride.

Suitable cationic monomers can include quaternary monomers of formula $-NR_3^+$, wherein each R can be identical or different, and can be a hydrogen atom, an alkyl group comprising 1 to 10 carbon atoms, or a benzyl group, optionally carrying a hydroxyl group, and including an anion (counter-ion). Examples of suitable anions include halides such as chlorides, bromides, sulphates, hydrosulphates, alkylsulphates (for example comprising 1 to 6 carbon atoms), phosphates, citrates, formates, and acetates.

Suitable cationic monomers can also include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride. Additional suitable cationic monomers can include trimethyl ammonium propyl (meth)acrylamido chloride.

Examples of monomers bearing a negative charge include alpha ethylenically unsaturated monomers including a phosphate or phosphonate group, alpha ethylenically unsaturated monocarboxylic acids, monoalkylesters of alpha ethylenically unsaturated dicarboxylic acids, monoalkylamides of alpha ethylenically unsaturated dicarboxylic acids, alpha ethylenically unsaturated compounds comprising a sulphonic acid group, and salts of alpha ethylenically unsaturated compounds comprising a sulphonic acid group.

Suitable monomers with a negative charge can include acrylic acid, methacrylic acid, vinyl sulphonic acid, salts of vinyl sulfonic acid, vinylbenzene sulphonic acid, salts of vinylbenzene sulphonic acid, alpha-acrylamidomethylpropanesulphonic acid, salts of alpha-acrylamidomethylpropanesulphonic acid, 2-sulphoethyl methacrylate, salts of 2-sulphoethyl methacrylate, acrylamido-2-methylpropanesulphonic acid (AMPS), salts of acrylamido-2-methylpropanesulphonic acid, and styrenesulphonate (SS).

Examples of nonionic monomers can include vinyl acetate, amides of alpha ethylenically unsaturated carboxylic acids, esters of an alpha ethylenically unsaturated monocarboxylic acids with an hydrogenated or fluorinated alcohol, polyethylene oxide (meth)acrylate (i.e. polyethoxylated (meth)acrylic acid), monoalkylesters of alpha ethylenically unsaturated dicarboxylic acids, monoalkylamides of alpha ethylenically unsaturated dicarboxylic acids, vinyl nitriles, vinylamine amides, vinyl alcohol, vinyl pyrolidone, and vinyl aromatic compounds.

Suitable nonionic monomers can also include styrene, acrylamide, methacrylamide, acrylonitrile, methylacrylate, ethylacrylate, n-propylacrylate, n-butylacrylate, methylmethacrylate, ethylmethacrylate, n-propylmethacrylate, n-butylmethacrylate, 2-ethyl-hexyl acrylate, 2-ethyl-hexyl methacrylate, 2-hydroxyethylacrylate and 2-hydroxyethylmethacrylate.

The anionic counterion ($X^-$) in association with the synthetic cationic polymers can be any known counterion so long as the polymers remain soluble or dispersible in water, in the personal care composition, or in a coacervate phase of the personal care composition, and so long as the counterions are physically and chemically compatible with the essential components of the personal care composition or do not otherwise unduly impair product performance, stability or aesthetics. Non limiting examples of suitable counterions can include halides (e.g., chlorine, fluorine, bromine, iodine), sulfate, and methylsulfate.

The cationic polymer described herein can also aid in repairing damaged hair, particularly chemically treated hair by providing a surrogate hydrophobic F-layer. The microscopically thin F-layer provides natural weatherproofing, while helping to seal in moisture and prevent further damage. Chemical treatments damage the hair cuticle and strip away its protective F-layer. As the F-layer is stripped away, the hair becomes increasingly hydrophilic. It has been found that when lyotropic liquid crystals are applied to chemically treated hair, the hair becomes more hydrophobic and more virgin-like, in both look and feel. Without being limited to any theory, it is believed that the lyotropic liquid crystal complex creates a hydrophobic layer or film, which coats the hair fibers and protects the hair, much like the natural F-layer protects the hair. The hydrophobic layer can return the hair to a generally virgin-like, healthier state. Lyotropic liquid crystals are formed by combining the synthetic cationic polymers described herein with the aforementioned anionic detersive surfactant component of the personal care composition. The synthetic cationic polymer has a relatively high charge density. It should be noted that some synthetic polymers having a relatively high cationic charge density do not form lyotropic liquid crystals, primarily due to their abnormal linear charge densities. Such synthetic cationic polymers are described in PCT Patent App. No. WO 94/06403. The synthetic polymers described herein can be formulated in a stable personal care composition that provides improved conditioning performance, with respect to damaged hair.

Cationic synthetic polymers that can form lyotropic liquid crystals have a cationic charge density of from about 2

meq/gm to about 7 meq/gm, and/or from about 3 meq/gm to about 7 meq/gm, and/or from about 4 meq/gm to about 7 meq/gm. The cationic charge density is about 6.2 meq/gm. The polymers also have a M. Wt. of from about 1,000 to about 5,000,000, and/or from about 10,000 to about 2,000,000, and/or from about 100,000 to about 2,000,000.

Cationic synthetic polymers that provide enhanced conditioning and deposition of benefit agents but do not necessarily form lytropic liquid crystals can have a cationic charge density of from about 0.7 meq/gm to about 7 meq/gm, and/or from about 0.8 meq/gm to about 5 meq/gm, and/or from about 1.0 meq/gm to about 3 meq/gm. The polymers also have a M.Wt. of from about 1,000 g/mol to about 5,000,000 g/mol, from about 10,000 g/mol to about 2,000,000 g/mol, and from about 100,000 g/mol to about 2,000,000 g/mol.

Cationic Cellulose Polymer

Suitable cationic polymers can be cellulose polymers. Suitable cellulose polymers can include salts of hydroxyethyl cellulose reacted with trimethyl ammonium substituted epoxide, referred to in the industry (CTFA) as Polyquaternium 10 and available from Dwo/ Amerchol Corp. (Edison, N.J., USA) in their Polymer LR, JR, and KG series of polymers. Other suitable types of cationic cellulose can include the polymeric quaternary ammonium salts of hydroxyethyl cellulose reacted with lauryl dimethyl ammonium-substituted epoxide referred to in the industry (CTFA) as Polyquaternium 24. These materials are available from Dow/ Amerchol Corp. under the tradename Polymer LM-200. Other suitable types of cationic cellulose can include the polymeric quaternary ammonium salts of hydroxyethyl cellulose reacted with lauryl dimethyl ammonium-substituted epoxide and trimethyl ammonium substituted epoxide referred to in the industry (CTFA) as Polyquaternium 67. These materials are available from Dow/ Amerchol Corp. under the tradename SoftCAT Polymer SL-5, SoftCAT Polymer SL-30, Polymer SL-60, Polymer SL-100, Polymer SK-L, Polymer SK-M, Polymer SK-MH, and Polymer SK-H.

Additional cationic polymers are also described in the CTFA Cosmetic Ingredient Dictionary, 3rd edition, edited by Estrin, Crosley, and Haynes, (The Cosmetic, Toiletry, and Fragrance Association, Inc., Washington, D.C. (1982).

Techniques for analysis of formation of complex coacervates are known in the art. For example, microscopic analyses of the compositions, at any chosen stage of dilution, can be utilized to identify whether a coacervate phase has formed. Such coacervate phase can be identifiable as an additional emulsified phase in the composition. The use of dyes can aid in distinguishing the coacervate phase from other insoluble phases dispersed in the composition. Additional details about the use of cationic polymers and coacervates are disclosed in U.S. Patent No. 9,272,164.

**D. Liquid Carrier**

As can be appreciated, personal care compositions can desirably be in the form of pourable liquid under ambient conditions. Inclusion of an appropriate quantity of a liquid carrier can facilitate the formation of a personal care composition having an appropriate viscosity and rheology. A personal care composition can include, by weight of the composition, about 20% to about 95%, by weight, of a liquid carrier, and about 60% to about 85%, by weight, of a liquid carrier.

A liquid carrier can be water, or can be a miscible mixture of water and organic solvent. A liquid carrier can be water with minimal or no significant concentrations of organic solvent, except as otherwise incidentally incorporated into the composition as minor ingredients of other essential or optional components. Suitable organic solvents can include water solutions of lower alkyl alcohols and polyhydric alcohols. Useful lower alkyl alcohols include monohydric alcohols having 1 to 6 carbons, such as ethanol and isopropanol. Exemplary polyhydric alcohols include propylene glycol, hexylene glycol, glycerin, and propane diol.

**Optional Components**

As can be appreciated, personal care compositions described herein can include a variety of optional components to tailor the properties and characteristics of the composition. As can be appreciated, suitable optional components are well known and can generally include any components which are physically and chemically compatible with the essential components of the personal care compositions described herein. Optional components should not otherwise unduly impair product stability, aesthetics, or performance. Individual concentrations of optional components can generally range from about 0.001% to about 10%, by weight of a personal care composition. Optional components can be further limited to components which will not impair the clarity of a translucent personal care composition.

Suitable optional components which can be included in a personal care composition can include co-surfactants, deposition aids, conditioning agents (including hydrocarbon oils, fatty esters, silicones), anti-dandruff agents, suspending agents, viscosity modifiers, dyes, nonvolatile solvents or diluents (water soluble and insoluble), pearlescent aids, foam boosters, pediculocides, pH adjusting agents, perfumes, preservatives, chelants, proteins, skin active agents, sunscreens, UV absorbers, and vitamins. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of non-limiting materials that can be added to the composition herein.

Co-Surfactants

One or more co-surfactants can be included in a personal care composition to enhance various properties of a personal care composition. For example, a co-surfactant can improve the production of lather, facilitate easier rinsing, or further mitigate the harshness on detersive surfactants on keratinous tissue. A co-surfactant further can also aid in producing lather having more desirable textures and volume. Suitable co-surfactants can be selected from any known surfactants suitable for personal care compositions including amphoteric, zwitterionic, cationic, and nonionic surfactants. When included, a co-surfactant can be included in a ratio with the detersive surfactant. For example, the ratio of the detersive surfactant to a co-surfactant can be about 1:20 to about 1:4, and alternatively a ratio of about 1:12 to about 1:7.
Alternatively, a co-surfactant can be included by weight percentage of the personal care composition. For example, a personal care composition can include a co-surfactant by weight of about 0.5% to about 10%, about 0.5% to about 5%, about 0.5% to about 3%, about 0.5% to about 2%, and about 0.5% to about 1.75%.

Conditioning Agents

A personal care composition can include a silicone conditioning agent. Suitable silicone conditioning agents can include volatile silicone, non-volatile silicone, or combinations thereof. If including a silicone conditioning agent, the agent can be included from about 0.01% to about 10%, by weight of the composition, from about 0.1% to about 8%, from about 0.1% to about 5%, and/or from about 0.2% to about 3%. Examples of suitable silicone conditioning agents, and optional suspending agents for the silicone, are described in U.S. Reissue Pat. No. 34,584, U.S. Patent No. 5,104,646, and U.S. Patent No. 5,106609. Suitable silicone conditioning agents can have a viscosity, as measured at 25° C, from about 20 centistokes ("csk") to about 2,000,000 csk, from about 1,000 csk to about 1,800,000 csk, from about 50,000 csk to about 1,500,000 csk, and from about 100,000 csk to about 1,500,000 csk.
The dispersed silicone conditioning agent particles can have a volume average particle diameter ranging from about 0.01 micrometer to about 50 micrometer. For small particle application to hair, the volume average particle diameters can range from about 0.01 micrometer to about 4 micrometer, from about 0.01 micrometer to about 2 micrometer, from about 0.01 micrometer to about 0.5 micrometer. For larger particle application to hair, the volume average particle diameters typically range from about 5 micrometer to about 125 micrometer, from about 10 micrometer to about 90 micrometer, from about 15 micrometer to about 70 micrometer, and/or from about 20 micrometer to about 50 micrometer.
Additional material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. 1989).
Silicone emulsions suitable for the personal care compositions described herein can include emulsions of insoluble polysiloxanes prepared in accordance with the descriptions provided in U.S. Patent No. 4,476,282 and U.S. Patent Application Publication No. 2007/0276087. Suitable insoluble polysiloxanes include polysiloxanes such as alpha, omega hydroxy-terminated polysiloxanes or alpha, omega alkoxy-terminated polysiloxanes having a molecular weight within the range from about 50,000 to about 500,000 g/mol. The insoluble polysiloxane can have an average molecular weight within the range from about 50,000 to about 500,000 g/mol. For example, the insoluble polysiloxane may have an average molecular weight within the range from about 60,000 to about 400,000; from about 75,000 to about 300,000; from about 100,000 to about 200,000; or the average molecular weight may be about 150,000 g/mol. The insoluble polysiloxane can have an average particle size within the range from about 30 nm to about 10 micron. The average particle size may be within the range from about 40 nm to about 5 micron, from about 50nm to about 1micron, from about 75 nm to about 500 nm, or about 100 nm, for example.
The average molecular weight of the insoluble polysiloxane, the viscosity of the silicone emulsion, and the size of the particle comprising the insoluble polysiloxane are determined by methods commonly used by those skilled in the art, such as the methods disclosed in Smith, A. L. The Analytical Chemistry of Silicones, John Wiley & Sons, Inc.: New York, 1991. For example, the viscosity of the silicone emulsion can be measured at 30 °C with a Brookfield viscosimeter with spindle 6 at 2.5 rpm. The silicone emulsion can further include an additional emulsifier together with the anionic surfactant.
Other classes of silicones suitable for the personal care compositions described herein can include i) silicone fluids, including silicone oils, which are flowable materials having viscosity less than about 1,000,000 csk as measured at 25 °C; ii) aminosilicones, which contain at least one primary, secondary or tertiary amine; iii) cationic silicones, which contain at least one quaternary ammonium functional group; iv) silicone gums; which include materials having viscosity greater or equal to 1,000,000 csk as measured at 25 °C; v) silicone resins, which include highly cross-linked polymeric siloxane systems; vi) high refractive index silicones, having refractive index of at least 1.46, and vii) mixtures thereof.
Alternatively, the personal care composition can be substantially free of silicones. As used herein, substantially free of silicones means from about 0 to about 0.2 wt. %.

Organic Conditioning Materials

The conditioning agent of the personal care compositions described herein can also include at least one organic conditioning material such as oil or wax, either alone or in combination with other conditioning agents, such as the silicones described above. The organic material can be non-polymeric, oligomeric or polymeric. The organic material can be in the form of an oil or wax and can be added in the personal care formulation neat or in a pre-emulsified form. Suitable examples of organic conditioning materials can include: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to about 2,000,000 including those with CTFA names PEG-200, PEG-400, PEG-600, PEG-1000, PEG-2M, PEG-7M, PEG-14M, PEG-45M and mixtures thereof.

Emulsifiers

A variety of anionic and nonionic emulsifiers can be used in the personal care composition of the present invention. The anionic and nonionic emulsifiers can be either monomeric or polymeric in nature. Monomeric examples include, by way of illustrating and not limitation, alkyl ethoxylates, alkyl sulfates, soaps, and fatty esters and their derivatives. Polymeric examples include, by way of illustrating and not limitation, polyacrylates , polyethylene glycols, and block copolymers and their derivatives. Naturally occurring emulsifiers such as lanolins, lecithin and lignin and their derivatives are also non-limiting examples of useful emulsifiers.

Chelating Agents

The personal care composition can also comprise a chelant. Suitable chelants include those listed in A E Martell & R M Smith, Critical Stability Constants, Vol. 1, Plenum Press, New York & London (1974) and A E Martell & R D Hancock, Metal Complexes in Aqueous Solution, Plenum Press, New York & London (1996). When related to chelants, the term "salts and derivatives thereof" means the salts and derivatives comprising the same functional structure (e.g., same chemical backbone) as the chelant they are referring to and that have similar or better chelating properties. This term include alkali metal, alkaline earth, ammonium, substituted ammonium (i.e. monoethanolammonium, diethanolammonium, triethanolammonium) salts, esters of chelants having an acidic moiety and mixtures thereof, in particular all sodium, potassium or ammonium salts. The term "derivatives" also includes "chelating surfactant" compounds, such as those exemplified in U.S. Pat. No. 5,284,972, and large molecules comprising one or more chelating groups having the same functional structure as the parent chelants, such as polymeric EDDS (ethylenediaminedisuccinic acid) disclosed in U.S. Pat. No. 5,747,440. U.S. Patent No. 5,284,972 and U.S. Patent No. 5,747,440. Suitable chelants can further include histidine.

Levels of an EDDS chelant or histidine chelant in the personal care compositions can be low. For example, an EDDS chelant or histidine chelant can be included at about 0.01%, by weight. Above about 10% by weight, formulation and/or human safety concerns can arise. The level of an EDDS chelant or histidine chelant can be at least about 0.01%, by weight, at least about 0.05%, by weight, at least about 0.1%, by weight, at least about 0.25%, by weight, at least about 0.5%, by weight, at least about 1%, by weight, or at least about 2%, by weight, by weight of the personal care composition.

Gel Network

A personal care composition can also include a fatty alcohol gel network. Gel networks are formed by combining fatty alcohols and surfactants in the ratio of from about 1:1 to about 40:1, from about 2:1 to about 20:1, and/or from about 3:1 to about 10:1. The formation of a gel network involves heating a dispersion of the fatty alcohol in water with the surfactant to a temperature above the melting point of the fatty alcohol. During the mixing process, the fatty alcohol melts, allowing the surfactant to partition into the fatty alcohol droplets. The surfactant brings water along with it into the fatty alcohol. This changes the isotropic fatty alcohol drops into liquid crystalline phase drops. When the mixture is cooled below the chain melt temperature, the liquid crystal phase is converted into a solid crystalline gel network. Gel networks can provide a number of benefits to personal care compositions. For example, a gel network can provide a stabilizing benefit to cosmetic creams and hair conditioners. In addition, gel networks can provide conditioned feel benefits to hair conditioners and shampoos.

A fatty alcohol can be included in the gel network at a level by weight of from about 0.05%, by weight, to about 14%, by weight,. For example, the fatty alcohol can be included in an amount ranging from about 1%, by weight, to about 10%, by weight, and/or from about 6%, by weight, to about 8%, by weight,.

Suitable fatty alcohols include those having from about 10 to about 40 carbon atoms, from about 12 to about 22 carbon atoms, from about 16 to about 22 carbon atoms, and/or about 16 to about 18 carbon atoms. These fatty alcohols

can be straight or branched chain alcohols and can be saturated or unsaturated. Nonlimiting examples of fatty alcohols include cetyl alcohol, stearyl alcohol, behenyl alcohol, and mixtures thereof. Mixtures of cetyl and stearyl alcohol in a ratio of from about 20:80 to about 80:20 are suitable.

A gel network can be prepared by charging a vessel with water. The water can then be heated to about 74 °C. Cetyl alcohol, stearyl alcohol, and sodium laureth sulfate ("SLES") surfactant can then be added to the heated water. After incorporation, the resulting mixture can passed through a heat exchanger where the mixture is cooled to about 35 °C. Upon cooling, the fatty alcohols and surfactant crystallized can form crystalline gel network. Table 1 provides the components and their respective amounts for an example gel network composition.

**Table 1**

| Ingredient | Wt. % |
|---|---|
| Water | 78.27% |
| ta | 4.18% |
| Stearyl Alcohol | 7.52% |
| Sodium laureth-3 sulfate (28% Active) | 10.00% |
| 5-Chloro-2-methyl-4-isothiazolin-3-one, Kathon CG | 0.03% |

Benefit Agents

A personal care composition can further include one or more benefit agents. Exemplary benefit agents include, but are not limited to, particles, colorants, perfume microcapsules, gel networks, and other insoluble skin or hair conditioning agents such as skin silicones, natural oils such as sun flower oil or castor oil. The benefit agent can be selected from the group consisting of: particles; colorants; perfume microcapsules; gel networks; other insoluble skin or hair conditioning agents such as skin silicones, natural oils such as sun flower oil or castor oil; and mixtures thereof.

Suspending Agent

A personal care composition can include a suspending agent at concentrations effective for suspending water-insoluble material in dispersed form in the compositions or for modifying the viscosity of the composition. Such concentrations range from about 0.1% to about 10%, and from about 0.3% to about 5.0%, by weight of the compositions. As can be appreciated however, suspending agents may not be necessary when certain glyceride ester crystals are included as certain glyceride ester crystals can act as suitable suspending or structuring agents.

Suitable suspending agents can include anionic polymers and nonionic polymers. Useful herein are vinyl polymers such as cross linked acrylic acid polymers with the CTFA name Carbomer, cellulose derivatives and modified cellulose polymers such as methyl cellulose, ethyl cellulose, hydroxyethyl cellulose, hydroxypropyl methyl cellulose, nitro cellulose, sodium cellulose sulfate, sodium carboxymethyl cellulose, crystalline cellulose, cellulose powder, polyvinylpyrrolidone, polyvinyl alcohol, guar gum, hydroxypropyl guar gum, xanthan gum, arabia gum, tragacanth, galactan, carob gum, guar gum, karaya gum, carragheenin, pectin, agar, quince seed (Cydonia oblonga Mill), starch (rice, corn, potato, wheat), algae colloids (algae extract), microbiological polymers such as dextran, succinoglucan, pulleran, starch-based polymers such as carboxymethyl starch, methylhydroxypropyl starch, alginic acid-based polymers such as sodium alginate, alginic acid propylene glycol esters, acrylate polymers such as sodium polyacrylate, polyethylacrylate, polyacrylamide, polyethyleneimine, and inorganic water soluble material such as bentonite, aluminum magnesium silicate, laponite, hectonite, and anhydrous silicic acid.

Other suitable suspending agents can include crystalline suspending agents which can be categorized as acyl derivatives, long chain amine oxides, and mixtures thereof. Examples of such suspending agents are described in U.S. Patent No. 4,741,855. Suitable suspending agents include ethylene glycol esters of fatty acids having from 16 to 22 carbon atoms. The suspending agent can be an ethylene glycol stearates, both mono and distearate, but particularly the distearate containing less than about 7% of the mono stearate. Other suitable suspending agents include alkanol amides of fatty acids, having from about 16 to about 22 carbon atoms, alternatively from about 16 to about 18 carbon atoms, suitable examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); long chain esters of long chain alkanol amides (e.g., stearamide diethanolamide distearate, stearamide monoethanolamide stearate); and glyceryl esters as previously described. Long chain acyl derivatives, ethylene glycol esters of long chain carboxylic acids, long chain amine oxides, and alkanol amides of long chain carboxylic acids can also be used as suspending agents.

Other long chain acyl derivatives suitable for use as suspending agents include N,N-dihydrocarbyl amido benzoic acid and soluble salts thereof (e.g., Na, K), particularly N,N-di(hydrogenated) $C_{16}$, $C_{18}$ and tallow amido benzoic acid species of this family, which are commercially available from Stepan Company (Northfield, Ill., USA).

Examples of suitable long chain amine oxides for use as suspending agents include alkyl dimethyl amine oxides, e.g., stearyl dimethyl amine oxide.

Other suitable suspending agents include primary amines having a fatty alkyl moiety having at least about 16 carbon atoms, examples of which include palmitamine or stearamine, and secondary amines having two fatty alkyl moieties each having at least about 12 carbon atoms, examples of which include dipalmitoylamine or di(hydrogenated tallow)amine. Still other suitable suspending agents include di(hydrogenated tallow)phthalic acid amide, and crosslinked maleic anhydride-methyl vinyl ether copolymer.

Viscosity Modifiers

Viscosity modifiers can be used to modify the rheology of a personal care composition. Suitable viscosity modifiers can include Carbomers with tradenames Carbopol 934, Carbopol 940, Carbopol 950, Carbopol 980, and Carbopol 981, all available from B. F. Goodrich Company, acrylates/steareth-20 methacrylate copolymer with tradename ACRYSOL 22 available from Rohm and Hass, nonoxynyl hydroxyethylcellulose with tradename AMERCELL POLYMER HM-1500 available from Amerchol, methylcellulose with tradename BENECEL, hydroxyethyl cellulose with tradename NATROSOL, hydroxypropyl cellulose with tradename KLUCEL, cetyl hydroxyethyl cellulose with tradename POLYSURF 67, all supplied by Hercules, ethylene oxide and/or propylene oxide based polymers with tradenames CARBOWAX PEGs, POLYOX WASRs, and UCON FLUIDS, all supplied by Amerchol. Sodium chloride can also be used as a viscosity modifier. Other suitable rheology modifiers can include cross-linked acrylates, cross-linked maleic anhydride co-methylvinylethers, hydrophobically modified associative polymers, and mixtures thereof.

The personal care composition can have a viscosity of 1,000 cP to 20,000 cP, or from about 2,500 cP to about 12,000 cP, or from about 3,500 cP to about 8,500 cP, measured at 26.6 °C with a Brookfield R/S Plus Rheometer at 2 $s^{-1}$. cP means centipoises.

Dispersed Particles

Dispersed particles as known in the art can be included in a personal care composition. If including such dispersed particles, the particles can be incorporated, by weight of the composition, at levels of about 0.025% or more, about 0.05% or more, about 0.1% or more, about 0.25% or more, and about 0.5% or more. However, the personal care compositions can also contain, by weight of the composition, about 20% or fewer dispersed particles, about 10% or fewer dispersed particles, about 5% or fewer dispersed particles, about 3% or fewer dispersed particles, and about 2% or fewer dispersed particles.

As can be appreciated, a personal care composition can include still further optional components. For example, amino acids can be included. Suitable amino acids can include water soluble vitamins such as vitamins B1, B2, B6, B12, C, pantothenic acid, pantothenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine, tryptamine, and their salts.

Anti-dandruff agents can be included. As can be appreciated, the formation of a coacervate can facilitate deposition of the anti-dandruff agent to the scalp.

A personal care composition can optionally include pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. I. Names. The compositions can also include antimicrobial agents which are useful as cosmetic biocides and antidandruff agents including: water soluble components such as piroctone olamine, water insoluble components such as 3,4,4'- trichlorocarbanilide (trichlosan), triclocarban and zinc pyrithione.

One or more stabilizers can be included. For example, one or more of ethylene glycol distearate, citric, citrate, a preservative such as kathon, sodium chloride, sodium benzoate, and ethylenediaminetetraacetic acid ("EDTA") can be included to improve the lifespan of a personal care compositon.

**Method of Making a Personal Care Composition**

A personal care composition described herein can be formed similarly to known personal care compositions. For example, the process of making a personal care composition can include the step of mixing the surfactant, cationic polymer, glyceride ester, and liquid carrier together to form a personal care composition. A glyceride ester can be crystallized as a separate premix prior to addition to the other components of a personal care composition. The glyceride

ester can be crystallized as previously described.

## TEST METHODS

### A. Coacervate Quantity

Coacervate Centrifuge Method

The quantity of coacervate can be measured by centrifuging a diluted personal care composition and measuring coacervate gravimetrically. Herein, several different dilutions can be made in a 50 ml centrifuge tube. The dilution is mixed by placement on a rotator overnight to allow coacervate to form then centrifuged for 20 minutes at 9200 rpm using a Beckman Couller TJ25 centrifuge. The supernatant is then removed and the remaining settled coacervate assessed gravimetrically. % Coacervate is calculated as the weight of settled coacervate as a percentage of the weight of shampoo added to the centrifuge tube using the equation below. This quantifies the percentage of the shampoo the participates in the coacervate phase.

$$\%\,Coacervate = \frac{Weight\ of\ settled\ coacervate}{Weight\ of\ shampoo\ added\ to\ centrifuge\ tube} \times 100$$

### B. Glyceride Ester Fiber Characterization

Fiber Size

Glyceride ester dispersions (e.g., 1.5% Thixcin concentration or alternate structurant) are diluted 1:25. Using a light microscope with an optical camera at a magnifying power of 400x, 15 fibers are measured for length and width in the premix solutions. This test can also be performed on personal care compositions to detect fiber size.

Fiber Percentage

Using a light microscope with an optical camera at a magnifying power of 200X, 15 sample fields, with dimensions of 430um X 22mm, are viewed. All non-fiber particles within each of the samples are counted and measured. Due to the fact of the surfactant and Thixcin (or alternative structurant) have very similar densities, the % fibers in the samples are calculated using surface area using the equations below:

| | |
|---|---|
| Sample Area | = length x width of sample field |
| Total Fiber Area | = 1.5% (amount of glyceride ester in sample) x sample area |
| Non-fiber % | = Total non-fiber area / total fiber area |
| Fiber % | = 100 - Non-fiber % |

### C. Lather Characterization

Kruss DFA100 Lather Characterization

A shampoo dilution of 10 parts by weight water to 1 part by weight shampoo is prepared. The shampoo dilution is dispensed into the Kruss DFA100 which generates the lather and measures lather properties.

### D. Wet Combing Characterization

Wet Combing Force Method

Hair switches of 4 grams general population hair at 8 inches length are used for the measurement. Each hair switch is treated with 4 cycles (1 lather/rinse steps per cycle, 0.1gm shampoo/gm hair on each lather/rinse step, drying between each cycle) with the shampoo. Four switches are treated with each shampoo. The hair is not dried after the last treatment cycle. While the hair is wet, the hair is pulled through the fine tooth half of two Beautician 3000 combs. Force to pull the hair switch through the combs is measured by a friction analyzer (such as Instron or MTS tensile measurement) with a load cell and outputted in gram-force (gf). The pull is repeated for a total of five pulls per switch. Average wet combing

force is calculated by averaging the force measurement from the five pulls across the four hair switches treated with each shampoo. Data can be shown as average wet combing force through one or both of the two combs.

## EXAMPLES

The following Examples illustrate various personal care compositions and glyceride ester premix compositions. Each of the personal care compositions and glyceride ester premix compositions is prepared by conventional formulation and mixing techniques.

Tables 2 and 3 depict Examples of glyceride ester premix compositions. The glyceride ester crystals formed in Table 2 exhibit crystalline geometries which both increase coacervate formation and improve coacervate properties and additionally contribute stability to the personal care compositions. The glyceride ester crystals formed in Table 3 provide increased coacervate formation and improved properties but do not increase the stability of the personal care compositions and may also utilize supplemental stability providing entities such as structuring and suspending agents.

**TABLE 2**

| Ingredients | Example 1 | Example 2 | Example 3 | Example 4 | Example 5 |
|---|---|---|---|---|---|
| Hydrogenated Castor Oil ("HCO") [1] | 1.5% | 0.5% | 1.5% | 1.5% | 1.5% |
| Sodium Lauryl Sulfate ("SLS") (29% surfactant in water) | 98.15% | 99.1% | 98.1% | NA | 98.13% |
| Citric Acid | 0.35% | 0.4% | 0.4% | NA | 0.37% |
| Ammonium Lauryl Sulfate ("ALS")(24% surfactant in water) | -- | -- | -- | 98.5% | -- |
| pH | 11 | 6.8 | 6.9 | 5.7 | 5.68 |
| Target Temperature | 80 °C | 75 °C | 80 °C | 80 °C | 80 °C |
| Above/Below Melting temperature of HCO | Below | Below | Below | Below | Below |
| Does any step in the process involve a clear solution of the structurant? | No | No | No | No | No |
| Cooling Rate | 2.5 °C /min | 2.5 °C /min | 2.5 °C /min | 2.5 °C/min | 1800 °C/min |
| Shear Device | Cowles Blade 300 rpm for 20 minutes | Cowles Blade 300 rpm for 20 minutes | Cowles Blade 300 rpm for 20 minutes | Cowles Blade 300 rpm for 20 minutes | Quadro |
| Description | Majority of crystals observed were non-aggregated fibers of uniform size | Majority of crystals observed were non-aggregated fibers of uniform size | Majority of crystals observed were non-aggregated fibers of uniform size | Majority of crystals observed were non-aggregated fibers of uniform size | Majority of crystals observed were non-aggregated fibers of uniform size |

(continued)

| Ingredients | Example 1 | Example 2 | Example 3 | Example 4 | Example 5 |
|---|---|---|---|---|---|
| Length of Fibers | Average= 28.9 micrometers Standard Deviation= 7.9 | -- | -- | -- | -- |
| Yield Stress | -- | 0.0784 Pascals | -- | 0.1430 Pascals | -- |
| [1] Thixcin R supplied by Elementis. | | | | | |

Example 1

An amount of 7.5 g of HCO is dispersed in a solution of 1.75 g of citric acid and 491 g of SLS until no large agglomerates are visible. Dispersion is accomplished by using a Cowles blade at 300 rpm for 20 minutes. After dispersion, the mixture is heated to target temperature of 80 °C and held for 5-20 minutes. After temperature hold, the mixture is cooled at a rate of 2.5 °C /min to 30 °C under low shear.

Example 2

An amount of 2.5 g of HCO is dispersed in a solution of 2.0 g of citric acid and 495.5 g of SLS until no large agglomerates are visible. Dispersion is accomplished by using a Cowles blade at 300 rpm for 20 minutes. After dispersion, the mixture is heated to target temperature of 75 °C and held for 5-20 minutes. After temperature hold, the mixture is cooled at a rate of 2.5 °C /min to 30 °C under low shear.

Example 3

An amount of 7.5 g of HCO is dispersed in a solution of 2.0 g of citric acid and 491 g of SLS until no large agglomerates are visible. Dispersion is accomplished by using a Cowles blade at 300 rpm for 20 minutes. After dispersion, the mixture is heated to target temperature of 80 °C and held for 15-30 minutes. After temperature hold, the mixture is cooled at a rate of 2.5 °C /min to 30 °C under low shear.

Example 4

An amount of 7.5 g of HCO is dispersed in 491 g of ALS until no large agglomerates are visible. Dispersion is accomplished by using a Cowles blade at 300 rpm for 20 minutes. After dispersion, the mixture is heated to target temperature of 80 °C and held for 5-20 minutes. After temperature hold, the mixture is cooled at a rate of 2.5 °C /min to 30 °C under low shear.

Example 5

An amount of 3.6 Kg of HCO is dispersed in a solution of 0.888 Kg of citric acid and 235.4 Kg of SLS. Dispersion is accomplished by using a quadro. After dispersion, the mixture is heated to target temperature of 80 °C and held for 5-20 minutes. After temperature hold, the mixture is pumped through a cooling device into another vessel.

**TABLE 3**

| Ingredients | Example 6 | Example 7 | Example 8 | Example 9 |
|---|---|---|---|---|
| Hydrogenated Castor Oil ("HCO")[1] | 1.75% | 1.75% | 0.39% | 0.39% |
| Sodium Lauryl Sulfate ("SLS") (29% surfactant in water) | 97.85% | 97.85% | -- | -- |

(continued)

| Ingredients | Example 6 | Example 7 | Example 8 | Example 9 |
|---|---|---|---|---|
| Sodium Laureth 1 Sulfate ("SLE1S") (26% surfactant in water) | -- | -- | -- | -- |
| Citric Acid | 0.4% | 0.4% | -- | -- |
| AE3[2] | -- | -- | 11.25% | -- |
| AE9[3] | -- | -- | -- | 11.25% |
| Water | QS | QS | 88.36% | 88.36% |
| pH | 6.7 | 6.8 | -- | 7.5 |
| Target Temperature | 63 °C | 88 °C | 84 °C | 84 °C |
| Above/Below Melting temperature of HCO | Below | Above | Below | Below |
| Does any step in the process involve a clear solution of the structurant? | No | No | Yes | Yes |
| Cooling Rate | 2.5 °C/min | 2.5 °C/min | 2.0 °C/min | 2.0 °C/min |
| Shear Device | Cowles Blade 300rpm for 20 minutes | Cowles Blade 300rpm for 20 minutes | Stir bar | Stir Bar |
| Description | Very few fiber crystals were observed. Most crystals observed were spherically or irregular in shape. | Mixture of three crystal habits (fibers, spheres, irregular) of equal proportions | Majority of the crystals are highly aggregated non-fiber (spherical, irregular) particles. The minority fiber crystals are also highly aggregated. | 20-30% are small fibers while the other material is highly aggregated particles with irregular shape. |
| Yield Stress | -- | 0.0102 Pascals | No measurable yield stress | -- |

[1] Thixcin R supplied by Elementis.
[2] Tomadol 25-3 supplied by Tomah Products
[3] Tergitol 15-s-9 supplied by Dow Chemical

Example 6

An amount of 8.75 g of HCO is dispersed in a solution of 2.0 g of citric acid and 489.25 g of SLS until no large agglomerates are visible. Dispersion is accomplished by using a Cowles blade at 300 rpm for 20 minutes. After dispersion, the mixture is heated to target temperature of 63 °C and held for 5-20 minutes. After temperature hold, the mixture is cooled at a rate of 2.5 °C /min to 20 °C under low shear.

Example 7

An amount of 8.75 g of HCO is dispersed in a solution of 2.0 g of citric acid and 489.25 g of SLS until no large agglomerates are visible. Dispersion is accomplished by using a Cowles blade at 300 rpm for 20 minutes. After dispersion, the mixture is heated to target temperature of 88 °C and held for 5-20 minutes. After temperature hold, the mixture is cooled

at a rate of 2.5 °C /min to 20 °C under low shear.

Example 8

An amount of 2.5 g of HCO is dissolved in 72.8 g of surfactant at 84 °C. After dissolution, the solution is added to 572 g of water at 65 °C being mixed at 250 RPM. The solution is mixed under these conditions for 10 minutes. Then the mixing speed is reduced to 175 RPM and solution is mixed for an additional 30 minutes. The solution is then cooled to room temperature at a rate of 2 °C /minute.

Example 9

An amount of 2.5 g of HCO is dissolved in 72.8 g of surfactant at 84 °C. After dissolution, the solution is added to 572 g of water at 65 °C being mixed at 250 RPM. The solution is mixed under these conditions for 10 minutes. Then the mixing speed is reduced to 175 RPM and solution is mixed for an additional 30 minutes. The solution is then cooled to room temperature at a rate of 2 °C /minute.

Table 4 depicts the components, by weight percentage, of two Example personal care compositions. Example 10 is an Inventive Example because it includes 0.06%, by weight, of trihydroxystearin and demonstrates increased quantity of coacervate and associated improved coacervate properties. Example 11 is a Comparative Example because it does not include trihydroxystearin and in comparison exhibits lower quantity of coacervate without the associated improved coacervate properties. Performance is evaluated by a panel of 10 experts who use the product at home under controlled conditions. Increased quantity of coacervate and associated coacervate properties is expressed by panelists as Example 10 having creamier lather with smaller bubbles and more coating of the individual hair strands during and after rinsing in comparison to Example 11 which does not include trihydroxystearin.

**TABLE 4 - Personal Care Compositions**

| Component | Example 10 | Comparable Example 11 |
|---|---|---|
| Sodium laureth sulfate | 6.5 | 6.5 |
| Sodium lauryl sulfate | 6.5 | 6.5 |
| Cocamidopropyl betaine | 2.1 | 2.1 |
| Cocamide monoethanolamine | 0.5 | 0.5 |
| Guar hydroxypropyltrimonium chloride[1] | 0.1 | 0.1 |
| Trihydroxystearin (Crystallized) | 0.06 | -- |
| PEG-60 almond glycerides | 0.1 | 0.1 |
| Linoleamidopropyl PG-dimonium chloride phosphate | 0.075 | 0.075 |
| Water-USP Purified, Preservatives[2], pH Adjusters[3], Viscosity Adjusters[4] | Q.S. to 100 | Q.S. to 100 |

[1] Obtained as Jaguar® Excel from Solvay S.A. ( Brussels, BE)
[2] Kathon, sodium benzoate, and tetrasodium EDTA
[3] Citric Acid and sodium citrate (to a pH of about 5 to about 6.5)
[4] Sodium chloride and sodium xylene sulfonate to a viscosity of about 3,500 cP to about 6,500 cP

Table 5 depicts the components, by weight percentage, of four Example personal care compositions. Example 12, 13, 14, 15 are Inventive Examples because these examples include 0.114%, 0.171%, 0.228% and 0.285%, by weight, of trihydroxystearin respectively and demonstrate increasing quantity of coacervate with increasing level of trihydroxystearin. Example 11 does not include trihydroxystearin and in comparison exhibits a lower quantity of coacervate. This is shown in Table 6 and Table 7. The quantity of coacervate is obtained via the Coacervate Centrifuge Method. A shampoo dilution of 3 parts by weight water to 1 part by weight shampoo and a shampoo dilution of 9 parts by weight water to 1 part by weight shampoo are used here to form coacervate and measure the quantity of coacervate as a percentage of the shampoo that participates in the coacervate phase. The percentage increase in quantity of coacervate formed is calculated using the first equation listed below. The ratio of coacervate quantity increase to trihydroxystearin level is calculated using the second equation listed below.

$$\text{Percentage Increase in Quantity of Coacervate formed} = \frac{\%\ \text{Coacervate with Trihydroxystearin} - \%\ \text{Coacervate without Trihydroxystearin}}{\%\ \text{Coacervate without Trihydroxystearin}} \times 100$$

$$\text{Ratio of Coacervate Quantity Increase to Trihydroxystearin Level} = \frac{\%\ \text{Coacervate with Trihydroxystearin} - \%\ \text{Coacervate without Trihydoxystearin}}{\text{Trihydroxystearin Level (weight percentage)}}$$

**TABLE 5 - Personal Care Compositions**

| Component | Example 12 | Example 13 | Example 14 | Example 15 |
|---|---|---|---|---|
| Sodium laureth sulfate | 6.5 | 6.5 | 6.5 | 6.5 |
| Sodium lauryl sulfate | 6.5 | 6.5 | 6.5 | 6.5 |
| Cocamidopropyl betaine | 2.1 | 2.1 | 2.1 | 2.1 |
| Cocamide monoethanolamine | 0.5 | 0.5 | 0.5 | 0.5 |
| Guar hydroxypropyltrimonium chloride[1] | 0.1 | 0.1 | 0.1 | 0.1 |
| Trihydroxystearin (Crystallized) | 0.114 | 0.171 | 0.228 | 0.285 |
| PEG-60 almond glycerides | 0.1 | 0.1 | 0.1 | 0.1 |
| Linoleamidopropyl PG-dimonium chloride phosphate | 0.075 | 0.075 | 0.075 | 0.075 |
| Water-USP Purified, Preservatives[2], pH Adjusters[3], Viscosity Adjusters[4] | Q.S. to 100 | Q.S. to 100 | Q.S. to 100 | Q.S. to 100 |

[1] Obtained as Jaguar® Excel from Solvay S.A. ( Brussels, BE)
[2] Kathon CG from Rohm and Haas Company (Des Moines, US), sodium benzoate, and tetrasodium EDTA
[3] Citric Acid and sodium citrate (to a pH of about 5 to about 6.5)
[4] Sodium chloride and sodium xylene sulfonate to a viscosity of about 3,500 cP to about 6,500 cP

**TABLE 6 - Coacervate Quantity at 9:1 Dilution via Coacervate Centrifuge Method**

| Product | Comparable Example 11 | Example 10 | Example 12 | Example 13 | Example 14 | Example 15 |
|---|---|---|---|---|---|---|
| % Coacervate at 9:1 Dilution | 2.6% | 4.1% | 6.2% | 11.6% | 12.0% | 9.6% |
| Percentage Increase in Quantity of Coacervate Formed (% higher) | N/A | 58% | 138% | 346% | 362% | 269% |
| Ratio of Coacervate Quantity Increase to Trihydroxystearin Level | N/A | 25:1 | 32:1 | 53:1 | 41:1 | 25:1 |

**TABLE 7 - Coacervate Quantity at 3:1 Dilution via Coacervate Centrifuge Method**

| Product | Comparable Example 11 | Example 10 | Example 12 | Example 13 | Example 14 | Example 15 |
|---|---|---|---|---|---|---|
| % Coacervate at 3:1 Dilution | 1.8% | 3.0% | 3.7% | 5.0% | 6.0% | 7.2% |

(continued)

| Product | Comparable Example 11 | Example 10 | Example 12 | Example 13 | Example 14 | Example 15 |
|---|---|---|---|---|---|---|
| Percentage Increase in Quantity of Coacervate Formed (% higher) | N/A | 67% | 106% | 178% | 233% | 300% |
| Ratio of Coacervate Quantity Increase to Trihydroxystearin Level | N/A | 20:1 | 17:1 | 19:1 | 18:1 | 19:1 |

Examples 10, 12, 13, 14 and 15 also demonstrate increasing lather creaminess with increasing level of trihydroxystearin. Example 11 does not include trihydroxystearin and in comparison exhibits less lather creaminess. Lather creaminess is characterized herein by the Krűss Dynamic Foam Analyzer (DFA100). Lather creaminess is characterized by high final bubble count with small final bubble size (measured as mean bubble area). Within the lather, coacervate is understood to be in the lamellae water phase layer between air bubbles. Increasing the quantitiy of coacervate phase that is present in this lamellae water phase layer leads to increased bubble count, smaller bubble size and increased creaminess. Therefore, higher lather creaminess measured here is indicative of higher coacervate quantity. This is shown in Table 8.

**TABLE 8 - Lather Characterization via Kruss DFA100**

| Product | Comparable Example 11 | Example 10 | Example 12 | Example 13 | Example 14 | Example 15 |
|---|---|---|---|---|---|---|
| Final Bubble Count per $mm^2$ | 92 | 110 | 177 | 166 | 176 | 245 |
| Final Mean Bubble Area ($\mu m^2$) | 12,519 | 9,141 | 5,819 | 6,715 | 6,547 | 4,076 |

Examples 10, 12, 13, 14 and 15 also demonstrate improving wet detangling performance with increasing level of trihydroxystearin. Example 11 does not include trihydroxystearin and in comparison exhibits poorer wet detangling performance. This is shown in Table 9 via the Wet Combing Force Method where a lower average wet combing force demonstrates improved wet detangling performance by showing less force to pull the hair switch through the comb. It can be appreciated that it is easier to detangle hair that requires less force to pull through a comb.

**TABLE 9 - Wet Combing Characterization via Wet Combing Force Method**

| | Comparable Example 11 | Example 10 | Example 12 | Example 13 | Example 14 | Example 15 |
|---|---|---|---|---|---|---|
| Average Wet Combing Force through One Comb (gf) | 197 | 175 | 171 | 155 | 158 | 164 |

Examples 10, 12, 13, 14 and 15 further demonstrates that translucent personal care compositions can be formed which exhibit increased quantities of coacervate and improved coacervate properties. When a sample of Example 10 is tested on a UV/Vis spectrometer, 69% of light at 400 nm transmitted through the sample. Example 12 has a light transmittance of 49% at 400 nm. Example 13 has a light transmittance of 33% at 400 nm. Example 14 has a light transmittance of 17% at 400 nm. Example 15 has a light transmittance of 9% at 400 nm. Example 11, without the trihydroxystearin crystals, has a light transmittance of 95% at 400 nm.

It will be appreciated that other modifications of the present disclosure are within the skill of those in the hair care formulation art can be undertaken without departing from the spirit and scope of this invention. All parts, percentages, and ratios herein are by weight unless otherwise specified. Some components may come from suppliers as dilute solutions. The levels given reflect the weight percent of the active material, unless otherwise specified. A level of perfume and/or preservatives may also be included in the following examples.

The dimensions and values disclosed herein are not to be understood as being strictly limited to the exact numerical values recited. Instead, unless otherwise specified, each such dimension is intended to mean both the recited

value and a functionally equivalent range surrounding that value. For example, a dimension disclosed as "40 mm" is intended to mean "about 40 mm."

## Claims

**1.** A personal care composition comprising:

one or more surfactants, the one or more surfactants comprising one or more anionic surfactants, amphoteric surfactants, and zwitterionic surfactants;

a cationic polymer; and
glyceride ester crystals, wherein the glyceride ester crystals comprise hydrogenated castor oil, wherein the glyceride ester crystals have a fibrous shape, and
wherein the percentage of the personal care composition that participates in the coacervate phase at a 9:1 dilution is from 30% to 600% higher compared to the personal care composition without glyceride ester crystals, as measured by the Coacervate Centrifuge Method as disclosed herein, wherein the 9:1 dilution means a dilution of 9 parts by weight water to 1 part by weight personal care composition;

wherein the personal care composition is obtained by a method of making a personal care composition, the method comprising:

mixing one or more surfactants, a cationic polymer, and water to form a first mixture, the one or more surfactants comprising one or more anionic surfactants, amphoteric surfactants, and zwitterionic surfactants; and
adding glyceride ester crystals to the first mixture to form a personal care composition, wherein the glyceride ester crystals comprise hydrogenated castor oil, wherein the glyceride ester crystals have a fibrous shape, and wherein the personal care composition forms a greater quantity of coacervate than the personal care composition formed without glyceride ester crystals;
preparing the hydrogenated castor oil prior to addition to the first mixture, the step of preparing the hydrogenated castor oil comprising:

forming a crystalline premix by combining, under high shear, from 0.30% to 4%, by weight, of a hydrogenated castor oil, from 15% to 40%, by weight, of a surfactant, and water;
heating the premix composition to a temperature of 65°C to 84°C and mixing for 5 minutes to 20 minutes;
cooling the premix composition to 20°C at a rate of 10°C per minute to 1°C per minute under low shear to form a crystalline premix comprising fiber like crystals of hydrogenated castor oil; and

wherein the crystalline premix is added to the first mixture to form the personal care composition.

**2.** The personal care composition according to claim 1, wherein the glyceride ester crystals comprise trihydroxystearin.

**3.** The personal care composition according to any preceding claims, wherein the composition has from 0% to 0.25% of additional structuring or suspending agents other than glyceride ester crystals, by weight of the composition, preferably wherein the composition has from 0% to 0.5% of additional structuring or suspending agents other than glyceride ester crystals, by weight of the composition.

**4.** The personal care composition according to any preceding claims, wherein the composition comprises from 0.01% to 1.5%, by weight, of the glyceride ester crystals by weight of the composition, preferably wherein the composition comprises 0.03% to 0.5%, by weight, of the glyceride ester crystals, by weight of the composition, preferably wherein the composition comprises 0.05% to 0.25%, by weight, of the glyceride ester crystals, by weight of the composition.

**5.** The personal care composition according to any preceding claims, wherein the cationic polymer comprises a cationic guar polymer, preferably wherein the cationic guar polymer has a weight average molecular weight of 2.5 million g/mol or less.

**6.** The personal care composition according to any preceding claims, wherein the composition comprises from 0% to 0.05% of additional cationic polymers, by weight of the composition.

**7.** The personal care composition according to any preceding claims, wherein the composition comprises from 0% to 0.2% of silicones, by weight of the composition.

**8.** The personal care composition according to any preceding claims, wherein the composition comprises from 0% to 0.1% or less of sulfate surfactants by weight of the composition, preferably wherein the one or more surfactants are selected from the group consisting of an isethionate, a sarcosinate, a sulfonate, a sulfosuccinate, a sulfoacetate, a glycinate, a glutamate, a phosphate ester, an amphoacetate, a taurate and combinations thereof.

**9.** The personal care composition according to any preceding claims, further comprising one or more non-ionic surfactants.

**10.** A method of making a personal care composition, the method comprising:

mixing one or more surfactants, a cationic polymer, and water to form a first mixture, the one or more surfactants comprising one or more anionic surfactants, amphoteric surfactants, and zwitterionic surfactants; and
adding glyceride ester crystals to the first mixture to form a personal care composition, wherein the glyceride ester crystals comprise hydrogenated castor oil, wherein the glyceride ester crystals have a fibrous shape, and wherein the personal care composition forms a greater quantity of coacervate than the personal care composition formed without glyceride ester crystals, wherein the quantity of coacervate is measured according to the Coacervate Centrifuge Method as disclosed herein;
preparing the hydrogenated castor oil prior to addition to the first mixture, the step of preparing the hydrogenated castor oil comprising:

forming a crystalline premix by combining, under high shear, from 0.30% to 4%, by weight, of a hydrogenated castor oil, from 15% to 40%, by weight, of a surfactant, and water;
heating the premix composition to a temperature of 65°C to 84°C and mixing for 5 minutes to 20 minutes;
cooling the premix composition to 20°C at a rate of 10°C per minute to 1°C per minute under low shear to form a crystalline premix comprising fiber like crystals of hydrogenated castor oil; and

wherein the crystalline premix is added to the first mixture to form the personal care composition.

**Patentansprüche**

**1.** Körperpflegezusammensetzung, umfassend:

ein oder mehrere Tenside, das eine oder die mehreren Tenside umfassend ein oder mehrere anionische Tenside, amphotere Tenside und zwitterionische Tenside;
ein kationisches Polymer; und
Glyceridesterkristalle, wobei die Glyceridesterkristalle gehärtetes Rizinusöl umfassen, wobei die Glyceridesterkristalle eine faserige Form aufweisen, und
wobei der Prozentsatz der Körperpflegezusammensetzung, der an der Koazervatphase bei einer Verdünnung von 9 : 1 teilnimmt, um 30 % bis 600 % höher ist, im Vergleich zu der Körperpflegezusammensetzung ohne Glyceridesterkristalle, wie durch das hierin offenbarte Koazervatzentrifugenverfahren gemessen, wobei die Verdünnung von 9 : 1 eine Verdünnung von 9 Gewichtsteilen Wasser zu 1 Gewichtsteil Körperpflegezusammensetzung bedeutet;
wobei die Körperpflegezusammensetzung durch ein Verfahren zum Herstellen einer Körperpflegezusammensetzung erhalten wird, das Verfahren umfassend:

Mischen eines oder mehrerer Tenside, eines kationischen Polymers und Wasser, um eine erste Mischung auszubilden, das eine oder die mehreren Tenside umfassend ein oder mehrere anionische Tenside, amphotere Tenside und zwitterionische Tenside; und
Zugeben von Glyceridesterkristallen zu der ersten Mischung, um eine Körperpflegezusammensetzung auszubilden, wobei die Glyceridesterkristalle gehärtetes Rizinusöl umfassen, wobei die Glyceridesterkristalle eine faserige Form aufweisen und wobei die Körperpflegezusammensetzung eine größere Menge an Koazervat als die Körperpflegezusammensetzung ausbildet, die ohne Glyceridesterkristalle ausgebildet ist;
Aufbereiten des gehärteten Rizinusöls vor der Zugabe zu der ersten Mischung, der Schritt des Aufbereitens

des gehärteten Rizinusöls umfassend:

Ausbilden einer kristallinen Vormischung durch Kombinieren, unter hoher Scherung, von, zu 0,30 Gew.-% bis 4 Gew.-%, einem gehärteten Rizinusöl, von, zu 15 Gew.-% bis 40 Gew.-%, einem Tensid und Wasser;
Erwärmen der Vormischungszusammensetzung auf eine Temperatur von 65 °C bis 84 °C und Mischen für 5 Minuten bis 20 Minuten;
Abkühlen der Vormischungszusammensetzung auf 20 °C bei einer Geschwindigkeit von 10 °C pro Minute bis 1 °C pro Minute unter geringer Scherung, um eine kristalline Vormischung auszubilden, umfassend faserartige Kristalle aus gehärtetem Rizinusöl; und
wobei die kristalline Vormischung zu der ersten Mischung zugegeben wird, um die Körperpflegezusammensetzung auszubilden.

**2.** Körperpflegezusammensetzung nach Anspruch 1, wobei die Glyceridesterkristalle Trihydroxystearin umfassen.

**3.** Körperpflegezusammensetzung nach einem der vorstehenden Ansprüche, wobei die Zusammensetzung zu 0 Gew.-% bis 0,25 Gew.-% der Zusammensetzung zusätzliche Struktur- oder Suspendiermittel, abgesehen von Glyceridesterkristallen, aufweist, vorzugsweise wobei die Zusammensetzung zu 0 Gew.-% bis 0,5 Gew.-% der Zusammensetzung zusätzliche Struktur- oder Suspendiermittel, abgesehen von Glyceridesterkristallen, aufweist.

**4.** Körperpflegezusammensetzung nach einem der vorstehenden Ansprüche, wobei die Zusammensetzung zu 0,01 Gew.-% bis 1,5 Gew.-% der Zusammensetzung die Glyceridesterkristalle umfasst, vorzugsweise wobei die Zusammensetzung zu 0,03 Gew.-% bis 0,5 Gew.-% der Zusammensetzung die Glyceridesterkristalle umfasst, vorzugsweise wobei die Zusammensetzung zu 0,05 Gew.-% bis 0,25 Gew.-% der Zusammensetzung die Glyceridesterkristalle umfasst.

**5.** Körperpflegezusammensetzung nach einem der vorstehenden Ansprüche, wobei das kationische Polymer ein kationisches Guarpolymer umfasst, vorzugsweise wobei das kationische Guarpolymer ein durchschnittliches Molekulargewicht (Gewichtsmittel) von 2,5 Millionen g/mol oder weniger aufweist.

**6.** Körperpflegezusammensetzung nach einem der vorstehenden Ansprüche, wobei die Zusammensetzung zu 0 Gew.-% bis 0,05 Gew.-% der Zusammensetzung zusätzliche kationische Polymere umfasst.

**7.** Körperpflegezusammensetzung nach einem der vorstehenden Ansprüche, wobei die Zusammensetzung zu 0 Gew.-% bis 0,2 Gew.-% der Zusammensetzung Silikone umfasst.

**8.** Körperpflegezusammensetzung nach einem der vorstehenden Ansprüche, wobei die Zusammensetzung zu 0 Gew.-% bis 0,1 Gew.-% oder weniger der Zusammensetzung Sulfattenside umfasst, vorzugsweise wobei das eine oder die mehreren Tenside aus der Gruppe bestehend aus einer Isethionsäure, einem Sarcosinat, einem Sulfonat, einem Sulfosuccinat, einem Sulfoacetat, einem Glycinat, einem Glutamat, einem Phosphatester, einem Amphoacetat, einem Taurat und Kombinationen davon ausgewählt sind.

**9.** Körperpflegezusammensetzung nach einem der vorstehenden Ansprüche, ferner umfassend ein oder mehrere nichtionische Tenside.

**10.** Verfahren zum Herstellen einer Körperpflegezusammensetzung, das Verfahren umfassend:

Mischen eines oder mehrerer Tenside, eines kationischen Polymers und Wasser, um eine erste Mischung auszubilden, das eine oder die mehreren Tenside umfassend ein oder mehrere anionische Tenside, amphotere Tenside und zwitterionische Tenside; und
Zugeben von Glyceridesterkristallen zu der ersten Mischung, um eine Körperpflegezusammensetzung auszubilden, wobei die Glyceridesterkristalle gehärtetes Rizinusöl umfassen, wobei die Glyceridesterkristalle eine faserige Form aufweisen und wobei die Körperpflegezusammensetzung eine größere Menge an Koazervat ausbildet als die Körperpflegezusammensetzung, die ohne Glyceridesterkristalle ausgebildet ist, wobei die Menge an Koazervat nach dem hierin offenbarten Koazervatzentrifugenverfahren gemessen wird;
Aufbereiten des gehärteten Rizinusöls vor der Zugabe zu der ersten Mischung, der Schritt des Aufbereitens des gehärteten Rizinusöls umfassend:

Ausbilden einer kristallinen Vormischung durch Kombinieren, unter hoher Scherung, von, zu 0,30 Gew.-% bis 4 Gew.-%, einem gehärteten Rizinusöl, von, zu 15 Gew.-% bis 40 Gew.-%, einem Tensid und Wasser;
Erwärmen der Vormischungszusammensetzung auf eine Temperatur von 65 °C bis 84 °C und Mischen für 5 Minuten bis 20 Minuten;
Abkühlen der Vormischungszusammensetzung auf 20 °C bei einer Geschwindigkeit von 10 °C pro Minute bis 1 °C pro Minute unter geringer Scherung, um eine kristalline Vormischung auszubilden, umfassend faserartige Kristalle aus gehärtetem Rizinusöl; und
wobei die kristalline Vormischung zu der ersten Mischung zugegeben wird, um die Körperpflegezusammensetzung auszubilden.

## Revendications

**1.** Composition de soins personnels comprenant :

un ou plusieurs agents tensioactifs, le ou les agents tensioactifs comprenant un ou plusieurs agents tensioactifs anioniques, agents tensioactifs amphotères et agents tensioactifs zwitterioniques ;
un polymère cationique ; et
des cristaux d'ester de glycéride, dans laquelle les cristaux d'ester de glycéride comprennent de l'huile de ricin hydrogénée, dans laquelle les cristaux d'ester de glycéride ont une forme fibreuse, et
dans laquelle le pourcentage de la composition de soins personnels qui participe dans la phase de coacervats à une dilution de 9:1 est de 30 % à 600 % plus élevé par comparaison avec la composition de soins personnels sans cristaux d'ester de glycéride, tel que mesuré par le procédé de centrifugation de coacervat tel que décrit ici, dans laquelle la dilution de 9:1 désigne une dilution de 9 parties en poids d'eau pour 1 partie en poids de composition de soins personnels ;
dans laquelle la composition de soins personnels est obtenue par un procédé de fabrication d'une composition de soins personnels, le procédé comprenant :

le mélange d'un ou plusieurs agents tensioactifs, d'un polymère cationique et d'eau pour former un premier mélange, le ou les agents tensioactifs comprenant un ou plusieurs agents tensioactifs anioniques, agents tensioactifs amphotères et agents tensioactifs zwitterioniques ; et
l'ajout de cristaux d'ester de glycéride au premier mélange pour former une composition de soins personnels, dans laquelle les cristaux d'ester de glycéride comprennent de l'huile de ricin hydrogénée, dans laquelle les cristaux d'ester de glycéride ont une forme fibreuse, et dans laquelle la composition de soins personnels forme une plus grande quantité de coacervat que la composition de soins personnels formée sans cristaux d'ester de glycéride ;
la préparation de l'huile de ricin hydrogénée avant addition au premier mélange, l'étape de préparation de l'huile de ricin hydrogénée comprenant :

la formation d'un prémélange cristallin en combinant, sous cisaillement élevé, de 0,30 % à 4 %, en poids, d'une huile de ricin hydrogénée, de 15 % à 40 %, en poids, d'un agent tensioactif, et de l'eau ;
le chauffage de la composition de prémélange à une température de 65 °C à 84 °C et le mélange pendant 5 minutes à 20 minutes ;
le refroidissement de la composition de prémélange à 20 °C à une vitesse de 10 °C par minute à 1 °C par minute sous faible cisaillement pour former un prémélange cristallin comprenant des cristaux de type fibre d'huile de ricin hydrogénée ; et
dans laquelle le prémélange cristallin est ajouté au premier mélange pour former la composition de soins personnels.

**2.** Composition de soins personnels selon la revendication 1, dans laquelle les cristaux d'ester de glycéride comprennent de la trihydroxystéarine.

**3.** Composition de soins personnels selon de quelconques revendications précédentes, dans laquelle la composition a de 0 % à 0,25 % d'agents structurants ou de suspension supplémentaires autre que des cristaux d'ester de glycéride, en poids de la composition, de préférence dans laquelle la composition a de 0 % à 0,5 % d'agents structurants ou de suspension supplémentaires autres que des cristaux d'ester de glycéride, en poids de la composition.

**4.** Composition de soins personnels selon de quelconques revendications précédentes, dans laquelle la composition

comprend de 0,01 % à 1,5 %, en poids, des cristaux d'ester de glycéride en poids de la composition, de préférence dans laquelle la composition comprend 0,03 % à 0,5 %, en poids, des cristaux d'ester de glycéride, en poids de la composition, de préférence dans laquelle la composition comprend 0,05 % à 0,25 %, en poids, des cristaux d'ester de glycéride, en poids de la composition.

**5.** Composition de soins personnels selon de quelconques revendications précédentes, dans laquelle le polymère cationique comprend un polymère de gomme de guar cationique, de préférence dans laquelle le polymère de gomme de guar cationique a une masse moléculaire moyenne en poids de 2,5 millions g/mol ou moins.

**6.** Composition de soins personnels selon de quelconques revendications précédentes, dans laquelle la composition comprend de 0 % à 0,05 % de polymères cationiques supplémentaires, en poids de la composition.

**7.** Composition de soins personnels selon de quelconques revendications précédentes, dans laquelle la composition comprend de 0 % à 0,2 % de silicones, en poids de la composition.

**8.** Composition de soins personnels selon de quelconques revendications précédentes, dans laquelle la composition comprend de 0 % à 0,1 % ou moins d'agents tensioactifs sulfate en poids de la composition, de préférence dans laquelle le ou les agents tensioactifs sont choisis dans le groupe constitué d'un iséthionate, d'un sarcosinate, d'un sulfonate, d'un sulfosuccinate, d'un sulfoacétate, d'un glycinate, d'un glutamate, d'un ester de phosphate, d'un amphoacétate, d'un taurate et de combinaisons de ceux-ci.

**9.** Composition de soins personnels selon l'une quelconque des revendications précédentes, comprenant en outre un ou plusieurs agents tensioactifs non ioniques.

**10.** Procédé de fabrication d'une composition de soins personnels, le procédé comprenant :

le mélange d'un ou plusieurs agents tensioactifs, d'un polymère cationique et d'eau pour former un premier mélange, le ou les agents tensioactifs comprenant un ou plusieurs agents tensioactifs anioniques, agents tensioactifs amphotères et agents tensioactifs zwitterioniques ; et
l'ajout de cristaux d'ester de glycéride au premier mélange pour former une composition de soins personnels, dans lequel les cristaux d'ester de glycéride comprennent de l'huile de ricin hydrogénée, dans lequel les cristaux d'ester de glycéride ont une forme fibreuse, et dans lequel la composition de soins personnels forme une plus grande quantité de coacervat que la composition de soins personnels formée sans cristaux d'ester de glycéride, dans lequel la quantité de coacervat est mesurée selon le procédé de centrifugation de coacervat tel que décrit ici ;
la préparation de l'huile de ricin hydrogénée avant addition au premier mélange, l'étape de préparation de l'huile de ricin hydrogénée comprenant :

la formation d'un prémélange cristallin en combinant, sous cisaillement élevé, de 0,30 % à 4 %, en poids, d'une huile de ricin hydrogénée, de 15 % à 40 %, en poids, d'un agent tensioactif, et de l'eau ;
le chauffage de la composition de prémélange à une température de 65 °C à 84 °C et le mélange pendant 5 minutes à 20 minutes ;
le refroidissement de la composition de prémélange à 20 °C à une vitesse de 10 °C par minute à 1 °C par minute sous faible cisaillement pour former un prémélange cristallin comprenant des cristaux de type fibre d'huile de ricin hydrogénée ; et
dans lequel le prémélange cristallin est ajouté au premier mélange pour former la composition de soins personnels.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 81778610 A
- US 2016106663 A
- US 6649155 B
- US 20080317698
- US 20080206355
- US 9138429 B
- US 3332880 A
- US 2486921 A
- US 2486922 A
- US 2396278 A
- US 4565647 A
- US 2438091 A
- US 2528378 A
- US 2658072 A
- US 3929678 A
- US 5104646 A
- US 5106609 A
- WO 9406403 A
- US 9272164 B
- US 34584 A
- US 4476282 A
- US 20070276087
- US 5284972 A
- US 5747440 A
- US 4741855 A


### Non-patent literature cited in the description


- McCutcheon's, Emulsifiers and Detergents. M. C. Publishing Co., 1989
- **SOLAREK, D. B.** Cationic Starches in Modified Starches: Properties and Uses. CRC Press, Inc., 1986, 113-125
- **QIN-JI PENG** ; **ARTHUR S. PERLIN**. Observation on NMR Spectra of Starches in Dimethyl Sulfoxide, Iodine-Complexing, and Solvating in Water-Dimethyl Sulfoxide. *Carbohydrate Research*, 1987, vol. 160, 57-72
- **J. HOWARD BRADBURY** ; **J. GRANT COLLINS**. An Approach to the Structural Analysis of Oligosaccharides by NMR Spectroscopy. *Carbohydrate Research*, 1979, vol. 71, 15-25
- CTFA Cosmetic Ingredient Dictionary. The Cosmetic, Toiletry, and Fragrance Association, Inc., 1982
- The CTFA Cosmetic Ingredient Handbook. the Cosmetic, Toiletry, and Fragrance Association, Inc., 2004
- Encyclopedia of Polymer Science and Engineering. John Wiley & Sons, Inc., 1989, vol. 15, 204-308
- **SMITH, A. L.** The Analytical Chemistry of Silicones. John Wiley & Sons, Inc., 1991
- **A E MARTELL** ; **R M SMITH**. Critical Stability Constants. Plenum Press, 1974, vol. 1
- **A E MARTELL** ; **R D HANCOCK**. Metal Complexes in Aqueous Solution. Plenum Press, 1996